# EUROPEAN PATENT APPLICATION

(11) **EP 4 617 268 A1**
(43) Date of publication of application: **17.09.2025**
(21) Application number: 23888684.0
(22) Date of filing: 07.11.2023
(51) Int. Cl.: C07D 401/12, A61K 31/4709, A61K 31/506, A61P 9/00, A61P 25/02, C07D 403/12, C07D 405/12, C07D 405/14, C07D 409/12, C07D 409/14, C07D 413/12, C07D 417/12

(54) **QUINAZOLINE DERIVATIVES**

(30) Priority: 07.11.2022 JP 2022178402
(71) Applicant: Kyoto University, Kyoto-shi, Kyoto 606-8501 (JP)
(72) Inventor: HAGIWARA, Masatoshi, Kyoto 606-8501 (JP); AWAYA, Tomonari, Kyoto 606-8501 (JP); AJIRO, Masahiko, Kyoto 606-8501 (JP); GOTANDA, Kentoku, Tokyo 103-8426 (JP)
(74) Representative: Dehns
(86) International application number: PCT/JP2023/039985
(87) International publication number: WO 2024/101337

(57) **Abstract**

An aspect of the present disclosure provides a compound capable of suppressing aberrant splicing that contributes to the onset or progression of a disease, or a pharmaceutically acceptable salt thereof. An aspect of the present disclosure relates to a compound represented by Formula (I) below or a pharmaceutically acceptable salt thereof.

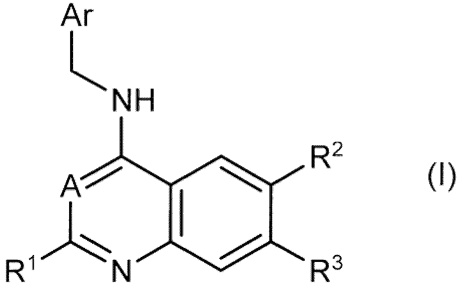

## Description

### Technical Field

The present disclosure relates to a compound capable of suppressing aberrant splicing that contributes to the onset or progression of a disease, or a pharmaceutically acceptable salt thereof.

### Background Art

RNA splicing refers to the process of removing introns from post-transcriptional pre-mRNA and joining exons together. In this process, exons are not only constitutively spliced, but the recognition of particular exon sequences or intron sequences may also be regulated in a fixed proportion depending on the tissue or cell lineage.

Even in an originally intron region, a genetic mutation can sometimes create a new splicing regulatory region, resulting in part of the intron region being recognized as an exon and causing aberrant splicing. This aberrant splicing may lead to various diseases.

Familial dysautonomia (FD) is one of the diseases caused by aberrant splicing. Familial dysautonomia is a congenital, fatal autosomal recessive genetic disease with impairment in sensory neurons and autonomic neurons due to abnormal neurodevelopment, neurodegeneration, and/or neurodeterioration. Most FD patients have a single nucleotide substitution (IVS20+6T>C mutation) in intron 20 of the IKBKAP (inhibitor of κ light polypeptide gene enhancer in B-cells, kinase complex-associated protein) gene. This mutation causes abnormal splicing (exon skipping) in which exon 20 is not incorporated into mRNA, mainly in nervous system tissues, resulting in the loss of normal IKBKAP protein production. This is thought to be the cause of the disease.

The present inventors have developed a splicing reporter technology in which different fluorescent proteins, such as GFP and RFP, are expressed depending on the selective use of an exon, and have constructed a splicing reporter that visualizes the aberrant splicing of IKBKAP, which is the causative gene for FD. Furthermore, the present inventors have elucidated the pathology of FD and found a low-molecular-weight compound (2-chloro-6-(2-furylmethyl)purine) that can correct abnormal splicing (see Patent Document 1, for example). They found that administration of this compound to cells from FD patients improves the disease condition, indicating that FD, which is an autosomal recessive genetic disease, can also be treated with pharmacological therapy.

In addition to FD, many other genetic diseases caused by aberrant splicing are known. Therefore, there is a need to develop new compounds that can suppress aberrant splicing.

### Prior Art Documents

### Patent Document

Patent Document 1: WO 2015/005491

### Non-Patent Document

Non-Patent Document 1: M. Ajiro et al., NATURE COMMUNICATIONS (2021)12:4507

### Disclosure of Invention

### Problem to be Solved by the Invention

The present disclosure provides a compound capable of suppressing aberrant splicing that contributes to the onset or progression of a disease, or a pharmaceutically acceptable salt thereof.

### Means for Solving Problem

An aspect of the present disclosure relates to a compound represented by Formula (I) below or a pharmaceutically acceptable salt thereof: 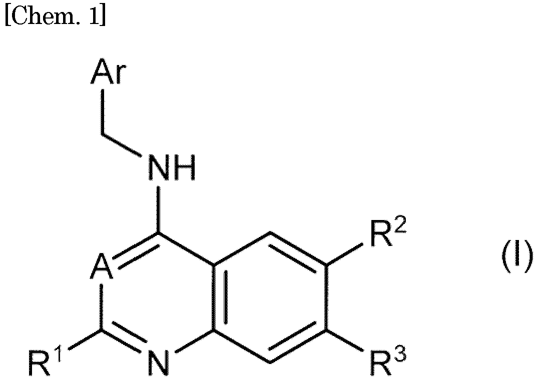 wherein, in Formula (I),
A represents CH or N;
R¹ represents a halogen atom;
R² and R³ are each independently selected from the group consisting of a hydrogen atom, -OR⁴, -O(CH₂)ₜR⁵, and -O(CH₂)ₜC(O)R⁵, where at least one of R² and R³ represents -OR⁴, -O(CH₂)ₜR⁵, or -O(CH₂)ₜC(O)R⁵, and t is 1, 2, 3, or 4,
   R⁴ representing a hydrogen atom or a C₁-C₆ alkyl group, and
   R⁵ being selected from the group consisting of a C₃-C₆ cycloalkyl group, a 4- to 10-membered heterocyclyl group, -NR⁶R⁷, and -OR⁶,
   R⁶ and R⁷ each independently representing a hydrogen atom or a C₁-Cs alkyl group; and
Ar is selected from the group consisting of a 5- to 10-membered heteroaryl group and a 6- to 12-membered aryl group, where the 5- to 10-membered heteroaryl group and the 6- to 12-membered aryl group may have one or more substituents.

Another aspect of the present disclosure relates to a pharmaceutical composition containing, as an active ingredient, the compound of the present disclosure or pharmaceutically acceptable salt thereof.

Another aspect of the present disclosure relates to a pharmaceutical composition for treating a genetic disease caused by aberrant splicing, the pharmaceutical composition containing, as an active ingredient, the compound of the present disclosure or pharmaceutically acceptable salt thereof.

Another aspect of the present disclosure relates to a pharmaceutical composition for treating a genetic disease caused by aberrant splicing, the pharmaceutical composition containing, as an active ingredient, a compound represented by Formula (I) above or a pharmaceutically acceptable salt thereof.

Another aspect of the present disclosure relates to a method for treating a genetic disease caused by aberrant splicing, the method including administering the compound of the present disclosure or pharmaceutically acceptable salt thereof to a subject.

Another aspect of the present disclosure relates to use of the compound of the present disclosure or pharmaceutically acceptable salt thereof in production of a pharmaceutical composition for use in treatment of a genetic disease caused by aberrant splicing.

### Effects of the Invention

The present disclosure can provide a compound capable of suppressing aberrant splicing that contributes to the onset or progression of a disease, or a pharmaceutically acceptable salt thereof.

### Brief Description of Drawings

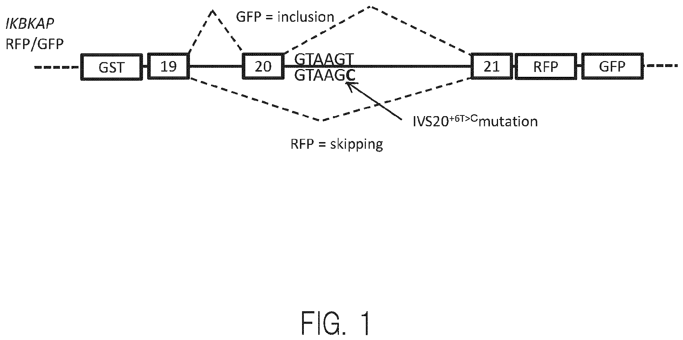

[FIG. 1] FIG. 1 is a diagram illustrating an example of the configuration of a SPREADD reporter system for a splicing mutation of the IKBKAP gene in familial dysautonomia.

### Description of the Invention

The term "halogen atom" as used in the present disclosure refers to a fluorine atom, a chlorine atom, a bromine atom, or an iodine atom, preferably a fluorine atom or a chlorine atom, and more preferably a chlorine atom.

The term "C₁-C₆ alkyl group" as used in the present disclosure refers to a linear or branched alkyl group having 1 to 6 carbon atoms. In one or more embodiments, examples of the C₁-C₆ alkyl group include a methyl group, an ethyl group, an n-propyl group, an isopropyl group, an n-butyl group, a sec-butyl group, a tert-butyl group, an n-pentyl group, an isopentyl group, a 2-methylbutyl group, a neopentyl group, a 1-ethylpropyl group, an n-hexyl group, a 4-methylpentyl group, a 3-methylpentyl group, a 2-methylpentyl group, a 1-methylpentyl group, a 3,3-dimethylbutyl group, a 2,2-dimethylbutyl group, a 1,1-dimethylbutyl group, a 1,2-dimethylbutyl group, a 1,3-dimethylbutyl group, a 2,3-dimethylbutyl group, a 2-ethylbutyl group, and the like.

The term "C₃-C₆ cycloalkyl group" as used in the present disclosure refers to cyclic alkyl groups (saturated hydrocarbon groups (rings)) having 3 to 6 carbon atoms. In one or more embodiments, examples of the C₃-C₆ cycloalkyl group include a cyclopropyl group, a cyclobutyl group, a cyclopentyl group, a cyclohexyl group, and the like.

The term "4- to 10-membered heterocyclyl group" as used in the present disclosure refers to a saturated or unsaturated monocyclic or polycyclic 4- to 10-membered cycloalkyl group having 1 to 3 heteroatoms selected from the group consisting of nitrogen, oxygen, and sulfur atoms in the ring. In one or more embodiments, the 4- to 10-membered heterocyclyl group has one oxygen atom, one sulfur atom, one nitrogen atom, one oxygen atom and one nitrogen atom, one sulfur atom and one nitrogen atom, two oxygen atoms, or two nitrogen atoms.

In one or more embodiments, examples of a heterocyclyl group include an oxiranyl group, a thiaranyl group, an aziridinyl group, an oxetanyl group, a thietanyl group, an azetidinyl group, a tetrahydrofuryl group, a tetrahydrothiophenyl group, a pyrrolidinyl group, a tetrahydropyranyl group, a pyranyl group, a tetrahydrothiopyranyl group, a thiopyranyl group, a piperidinyl group, an imidazolidinyl group, a 1,4-dioxanyl group, a 1,3-dioxolanyl group, a 1,3-dioxolyl group, a 1,1-dioxo-thietanyl group, a 1,4-oxathianyl group, a morpholinyl group, a thiomorpholinyl group, a 1,4-dithianyl group, a piperazinyl group, a 1,4-azathianyl group, an oxepanyl group, a thiepanyl group, an azepanyl group, an isothizolidinyl group, a 1,4-dioxepanyl group, a 1,4-oxathiepanyl group, a 1,4-oxaazepanyl group, a 1,4-dithiepanyl group, a 1,4-thieazepanyl group, a 1,4-azaphosphinanyl group, a 1,4-diazepanyl group, a 1,2-tetrahydrothiazin-2-yl group, a 1,3-tetrahydrothiazin-3-yl group, a tetrahydrothiadiazinyl group, a 1,2-tetrahydrodiazin-2-yl group, a 1,3-tetrahydrodiazin-1-yl group, a tetrahydroazepinyl group, a chromanyl group, a chromenyl group, an oxazolidinyl group, an isoxazolidinyl group, a 1,3-oxazolidin-3-yl group, an oxazinyl group, an isothiazolidinyl group, a 1,3-thiazolidin-3-yl group, a 1,2-pyrazolidin-2-yl group, a 1,3-pyrazolidin-1-yl group, a 7-oxa-1-aza-spiro[4.4]nonanyl group, a 3-azabicyclo[3.1.0]hexanyl group, an indolinyl group, a dihydroindolinyl group, an octahydro-1H-indolyl group, an octahydro-2H-pyrido[1,2-a]pyrazinyl group, a 3-azabicyclo[4.1.0]heptanyl group, a 3,4-dihydro-2H-pyranyl group, a 1,2,3,4-tetrahydropyridyl group, a 1,2,5,6-tetrahydropyridyl group, a tetrahydro-1H-benzo[d]azepinyl group, and the like.

In one or more embodiments, examples of the 4- to 10-membered heterocyclyl group include an oxetanyl group, a morpholinyl group, a thietanyl group, a 1,1-dioxo-thietanyl group, a 1,4-dioxanyl group, and the like.

The term "6- to 12-membered aryl group" as used in the present disclosure refers to monocyclic and polycyclic aromatic hydrocarbon ring systems having 6 to 12 carbon atoms. In one or more embodiments, examples of the 6- to 12-membered aryl group include aromatic hydrocarbon groups having 6 to 10 carbon atoms. In one or more embodiments, examples of the 6- to 12-membered aryl group include a phenyl group, a 1-naphthyl group, a 2-naphthyl group, an acenaphthylenyl group, an azulenyl group, and the like.

In the present disclosure, the 6- to 12-membered aryl group may be substituted with one or more substituents in one or more embodiments. In one or more embodiments, examples of the substituent include a halogen atom, -NO₂, -CN, an alkyl group, an aryl group, a heteroaryl group, a heterocyclyl group, -(CH₂)ₚNR^{b}R^{c}, - NR^{b}COR^{c}, -NR^{b}S(O)₂R^{c}, -(CH₂)ₚC(O)OR^{d}, -C(O)NR^{b}R^{c}, -C(O)R^{d}, -C(O)OR^{d}, -OR^{d}, and the like. In one or more embodiments, the alkyl group, the aryl group, the heteroaryl group, and the heterocyclyl group may be substituted. In one or more embodiments, R^{b}, R^{c}, and R^{d} each independently represent a hydrogen atom or an alkyl group having 1 to 6 carbon atoms that may be substituted. p is an integer of 0, 1, 2, or 3.

The term "5- or 10-membered heteroaryl group" as used in the present disclosure refers to a 5- or 10-membered heteroaryl group having one, two, or three heteroatoms selected from the group consisting of nitrogen, oxygen, and sulfur atoms in the ring. In one or more embodiments, examples of the heteroaryl group include a furyl group, a thienyl group, a pyrrolyl group, an oxazolyl group, an isoxazolyl group, a thiazolyl group, an isothiazolyl group, an oxadiazolyl group, a diazinylpyridyl group, a pyridyl group, a pyrimidinyl group, an imidazolyl group, a 1,2,3-triazolyl group, a 1,2,4-triazolyl group, a tetrazolyl group, a 1,3,5-oxadiazolyl group, a 1,2,4-oxadiazolyl group, a 1,2,3-oxadiazolyl group, a 1,3,5-thiadiazolyl group, a 1,2,3-thiadiazolyl group, a 1,2,4-thiadiazolyl group, a pyridazinyl group, a pyrazinyl group, a 1,2,3-triazinyl group, a pyrazolo[3,4-blpyridyl group, a cinnolinyl group, a pteridinyl group, a purinyl group, a 6,7-dihydro-5H-[1]pyrindinyl group, a benzo[b]thiophenyl group, a benzoxazolyl group, a benzothiazolyl group, a benzisothiazolyl group, a benzisoxazolyl group, a benzimidazolyl group, a benzofuryl group, an isobenzofuryl group, an isindolyl group, an indolyl group, an indolizinyl group, an indazolyl group, an isoquinolinyl group, a quinolinyl group, a phthalazinyl group, a quinoxalinyl group, a quinazolinyl group, an ibenzoxazinyl group, and the like. In one or more embodiments, examples of the 5- to 10-membered heteroaryl group include a furyl group, a thiophenyl group, a 1,3-thiazolyl group, a pyridyl group, a 1,3-oxazolyl group, a 1H-pyrrolyl group, and the like.

In the present disclosure, the 5- to 10-membered heteroaryl group may be substituted with one or more substituents in one or more embodiments. Examples of the substituent are as described above.

Suitable substituents for the compound of the present disclosure will be described below.

In one or more embodiments, R¹ preferably represents a chlorine atom.

In one or more embodiments, R² and R³ each independently represent a hydrogen atom, -OR⁴, -O(CH₂)ₜR⁵, or -O(CH₂)ₜC(O)R⁵, and preferably a hydrogen atom, -OR⁴, or -O(CH₂)ₜR⁵. In one or more embodiments, R⁴ represents a hydrogen atom or a C₁-C₃ alkyl group, and preferably a methyl group, an ethyl group, a propyl group, or an isopropyl group. In one or more embodiments, R⁵ represents -NR⁶R⁷, -OH, a methyl group, a cyclopropyl group, an oxetanyl group, a morpholinyl group, a 1,1-dioxo-thietanyl group, or a dioxanyl group, and R⁶ and R⁷ each independently represent a hydrogen atom or a methyl group. In one or more embodiments, R⁵ represents an amino group, a methylamino group, a dimethylamino group, a hydrogen atom, a cyclopropyl group, an oxetanyl group, a morpholinyl group, a 1,1-dioxo-thietanyl group, or a dioxanyl group. In one or more embodiments, t is 1 or 2.

In one or more embodiments, R² represents a hydrogen atom, a hydroxy group, a methoxy group (-OCH₃), an ethoxy group, a propyloxy group, an isopropyloxy group, a methoxyethoxy group, a cyclopropylmethoxy group, a 3-oxetanylmethoxy group, a 3-oxetanylethoxy group, a 2-morpholinylethoxy group, a 2-morpholinylpropoxy group, a 1,1-dioxo-thietanylmethoxy group, a 1,1-dioxo-thietanylethoxy group, an aminoethoxy group, a (methylamino)ethoxy group, a (dimethylamino)ethoxy group, a hydroxyethoxy group, a 1,4-dioxanylmethoxy group, a carboxyethoxy group, a methoxycarbonylethoxy group, or a methoxycarbonylmethoxy group.

In one or more embodiments, R³ represents a hydrogen atom, a methoxy group (-OCH₃), an ethoxy group, an aminoethoxy group, or a (methylamino)ethoxy group, and preferably a methoxy group.

In one or more embodiments, Ar represents a 5- or 6-membered heteroaryl group, preferably a furyl group, a thiophenyl group, a pyrrolyl group, a thiazolyl group, an oxazolyl group, or a pyridyl group, more preferably a furyl group, a thiophenyl group, or a pyridyl group, and even more preferably a 2-furyl group.

In one or more embodiments, in the compound represented by Formula (I), Ar represents a 2-furyl group, R² represents a hydrogen atom, -O(CH₂)₂CH₃, -OCH₂R⁵, or -O(CH₂)₂R⁵, and R³ represents a methoxy group, where R⁵ represents -NR⁶R⁷, -OH, or an oxetanyl group, where R⁶ and R⁷ each independently represent a hydrogen atom or a methyl group. In the compound represented by Formula (I), preferably, Ar represents a 2-furyl group, R² represents a hydrogen atom, a methoxy group, an ethoxy group, a propyloxy group, an isopropyloxy group, a cyclopropylmethoxy group, a 3-oxetanylmethoxy group, an aminoethoxy group, a (methylamino)ethoxy group, a (dimethylamino)ethoxy group, a hydroxyethoxy group, or a 1,4-dioxanylmethoxy group, and R³ represents a methoxy group. In the compound represented by Formula (I), more preferably, Ar represents a 2-furyl group, R² represents a hydrogen atom, a propyloxy group, an aminoethoxy group, a (methylamino)ethoxy group, a 3-oxetanylmethoxy group, a (dimethylamino)ethoxy group, or a hydroxyethoxy group, and R³ represents a methoxy group.

In one or more embodiments, it is preferable that the compound of the present disclosure is a compound or a pharmaceutically acceptable salt thereof, the compound being any one compound selected from the group consisting of:
2-chloro-N-(furan-2-ylmethyl)-6,7-dimethoxyquinazolin-4-amine,
2-chloro-6,7-dimethoxy-N-(thiophen-2-ylmethyl)quinazolin-4-amine,
2-chloro-6,7-dimethoxy-N-(thiophen-3-ylmethyl)quinazolin-4-amine,
2-chloro-6,7-dimethoxy-N-(1,3-thiazol-2-ylmethyl)quinazolin-4-amine,
2-chloro-6, 7-dimethoxy-N-(pyridin-4-ylmethyl)quinazolin-4-amine,
2-chloro-6,7-dimethoxy-N-(1,3-oxazol-2-ylmethyl)quinazolin-4-amine,
2-chloro-6,7-dimethoxy-N-(1H-pyrrol-3-ylmethyl)quinazolin-4-amine,
2-chloro-N-(furan-3-ylmethyl)-6,7-dimethoxyquinazolin-4-amine,
2-chloro-6, 7-dimethoxy-N-(pyridin-2-ylmethyl)quinazolin-4-amine,
2-chloro-N-(furan-2-ylmethyl)-7-methoxyquinazolin-4-amine,
2-chloro-N-(furan-2-ylmethyl)-6-methoxyquinazolin-4-amine,
2-chloro-N-(furan-2-ylmethyl)-7-methoxyquinolin-4-amine,
2-chloro-N-(furan-2-ylmethyl)-6-methoxyquinolin-4-amine,
2-chloro-6-ethoxy-N-(furan-2-ylmethyl)-7-methoxyquinazolin-4-amine,
2-chloro-6-ethoxy-N-(furan-2-ylmethyl)-7-methoxyquinazolin-4-amine,
2-chloro-N-(furan-2-ylmethyl)-7-methoxy-6-propoxyquinazolin-4-amine,
2-chloro-N-(furan-2-ylmethyl)-7-methoxy-6-(propan-2-yloxy)quinazolin-4-amine,
2-chloro-N-(furan-2-ylmethyl)-7-methoxy-6-(2-methoxyethoxy)quinazolin-4-amine,
2-chloro-6-(cyclopropylmethoxy)-N-(furan-2-ylmethyl)-7-methoxyquinazolin-4-amine,
2-chloro-N-(furan-2-ylmethyl)-7-methoxy-6-(oxetan-3-ylmethoxy)quinazolin-4-amine,
2-chloro-N-(furan-2-ylmethyl)-7-methoxy-6-[2-(morpholin-4-yl)ethoxy] quinazolin-4-amine,
2-chloro-N-(furan-2-ylmethyl)-7-methoxy-6-[3-(morpholin-4-yl)propoxy]quinazolin-4-amine,
2-chloro-6-[2-(1,1-dioxidothiethan-3-yl)ethoxy]-N-(furan-2-ylmethyl)-7-methoxyquinazolin-4-amine,
6-(2-aminoethoxy)-2-chloro-N-(furan-2-ylmethyl)-7-methoxyquinazolin-4-amine,
2-chloro-N-(furan-2-ylmethyl)-7-methoxy-6-[2-(methylamino)ethoxy]quinazolin-4-amine,
2-chloro-6-[2-(dimethylamino)ethoxy]-N-(furan-2-ylmethyl)-7-methoxyquinazolin-4-amine,
2-({2-chloro-4-[(furan-2-ylmethyl)amino]-7-methoxyquinazolin-6-yl}oxy)ethanol,
2-chloro-6-(1,4-dioxan-2-ylmethoxy)-N-(furan-2-ylmethyl)-7-methoxyquinazolin-4-amine,
3-({2-chloro-4-[(furan-2-ylmethyl)amino]-7-methoxyquinazolin-6-yl}oxy)propanoic acid,
methyl 3-({2-chloro-4-[(furan-2-ylmethyl)amino]-7-methoxyquinazolin-6-yl}oxy)propanoate,
methyl ({2-chloro-4-[(furan-2-ylmethyl)amino]-7-methoxyquinazolin-6-yl}oxy)acetate,
7-(2-aminoethoxy)-2-chloro-N-(furan-2-ylmethyl)-6-methoxyquinazolin-4-amine,
2-chloro-4-[(furan-2-ylmethyl)aminol-6-methoxyquinazolin-7-ol,
2-chloro-N-(furan-2-ylmethyl)-6-methoxy-7-[2-(methylamino)ethoxy]quinazolin-4-amine, and
2-chloro-7-ethoxy-N-(furan-2-ylmethyl)-6-methoxyquinazolin-4-amine.

The term "pharmaceutically acceptable salt" as used in the present disclosure refers to a salt that can be used as a medicine, and may be a basic salt, an acid salt, or the like in one or more embodiments.

In one or more embodiments, examples of the "basic salt" include alkali metal salts, such as sodium salts, potassium salts, and lithium salts; alkaline earth metal salts, such as magnesium salts and calcium salts; organic base salts, such as N-methylmorpholine salts, triethylamine salts, tributylamine salts, diisopropylethylamine salts, dicyclohexylamine salts, N-methylpiperidine salts, pyridine salts, 4-pyrrolidinopyridine salts, and picoline salts; amino acid salts, such as glycine salts, lysine salts, arginine salts, ornithine salts, glutamates, and aspartates; and the like. Alkali metal salts are preferable.

In one or more embodiments, examples of the "acid salt" include hydrogen halide salts, such as hydrofluorides, hydrochlorides, hydrobromides, and hydroiodides; inorganic acid salts, such as nitrates, perchlorates, sulfates, and phosphates; lower alkane sulfonates, such as methanesulfonates, trifluoromethanesulfonates, and ethanesulfonates; arylsulfonates, such as benzenesulfonates and p-toluenesulfonates; organic acid salts, such as acetates, malates, fumarates, succinates, citrates, ascorbates, tartrates, oxalates, and maleates; amino acid salts, such as glycine salts, lysine salts, arginine salts, ornithine salts, glutamates, and aspartates; and the like. Hydrogen halide salts (in particular, hydrochlorides) are preferable.

A pharmaceutically acceptable salt of the compound of the present disclosure may encompass a hydrate. Also, the term "salt of a compound" as used in the present disclosure may encompass a solvate that may be formed as a result of the compound absorbing another certain type of solvent.

The compound of the present disclosure, pharmaceutically acceptable salt thereof, or solvate thereof may exist as various isomers, such as geometric isomers (cis- and trans-isomers, etc.), optical isomers (enantiomers) (tautomers, rotamers, D-isomers and L-isomers, etc.), and diastereomers, depending on the kind and the combination of substituents. Unless otherwise specified, the compound of the present disclosure should be construed as encompassing all such isomers, stereoisomers, and mixtures of these isomers and stereoisomers in any ratio. Mixtures of these isomers can be separated using a known dividing means.

The compound of the present disclosure also encompasses a labeled compound, that is, a compound in which one or two or more atoms of the compound are replaced with an isotope (e.g., ²H, ³H, ¹³C, ¹⁴C, ³⁵S, etc.).

A compound that is converted into a compound represented by Formula (I), which is an active ingredient of the pharmaceutical composition of the invention, through a reaction with an enzyme, gastric acid, or the like under *in vivo* physiological conditions, or in other words, a compound that is enzymatically oxidized, reduced, or hydrolyzed, for example, to a compound represented by Formula (I), or a compound that is hydrolyzed, for example, by gastric acid or the like to a compound represented by Formula (I), is encompassed by the present disclosure as a "medicinally acceptable prodrug compound". In one or more embodiments, a prodrug is a compound having a group that can be converted into an amino group, a hydroxy group, a carboxyl group, or the like of a compound by hydrolysis or under physiological conditions, and groups that form such prodrugs are described in Prog. Med, Vol. 5, pp. 2157-2161, 1985, for example.

When the compound represented by Formula (I) contains an amino group, compounds in which the amino group is acylated, alkylated, or phosphorylated (e.g., compounds in which the amino group is eicosanoylated, alanylated, pentylaminocarbonylated, (5-methyl-2-oxo-1,3-dioxolen-4-yl)methoxycarbonylated, tetrahydrofurylated, pyrrolidylmethylated, pivaloyloxymethylated, or tert-butylated) can be presented as examples of the prodrug. When the compound represented by Formula (I) contains a hydroxy group, compounds in which the hydroxy group is acylated, alkylated, phosphorylated, or a borylated (e.g., compounds in which the hydroxy group is acetylated, palmitoylated, propanoylated, pivaloylated, succinylated, fumarylated, alanylated, or dimethylaminomethylcarbonylated) can be presented as examples of the prodrug. When the compound represented by Formula (I) contains a carboxy group, compounds in which the carboxy group is esterified or amidated (e.g., compounds in which the carboxy group is ethyl esterified, phenyl esterified, carboxymethyl esterified, dimethylaminomethyl esterified, pivaloyloxymethyl esterified, ethoxycarbonyloxyethyl esterified, or methylamidated) can be presented as examples of the prodrug.

### Production Methods

Hereinafter, representative methods for producing a compound represented by Formula (I) will be described. In one or more embodiments, the compound of the present disclosure can be produced using any one of the following methods A to E. Note that the production methods described below are merely examples, and the present disclosure should not be construed as being limited to these methods.

Compounds produced using the methods A to E below may be isolated and purified as non-solvates, salts thereof, or various solvates such as hydrates. The salts can be produced using ordinary methods. In one or more embodiments, examples of the salts include hydrochlorides, sulfates, and the like, as well as organic amine salts, sodium salts, potassium salts, and the like.

There are no particular limitations on solvents used in the reactions in steps of the methods A to E described below, as long as they partially dissolve the starting materials without inhibiting the reactions. In one or more embodiments, examples of the solvents include aliphatic hydrocarbons, aromatic hydrocarbons, halogenated hydrocarbons, ethers, ketones, esters, nitriles, carboxylic acids, alcohols, amides, sulfoxides, water, mixtures thereof, and the like.

In one or more embodiments, examples of the aliphatic hydrocarbons include n-hexane, n-pentane, petroleum ether, cyclohexane, and the like.

In one or more embodiments, examples of the aromatic hydrocarbons include benzene, toluene, xylene, and the like.

In one or more embodiments, examples of the halogenated hydrocarbons include dichloromethane (methylene chloride), chloroform, carbon tetrachloride, dichloroethane, chlorobenzene, dichlorobenzene, and the like.

In one or more embodiments, examples of the ethers include diethyl ether, diisopropyl ether, tetrahydrofuran (THF), dioxane, dimethoxyethane, diethylene glycol dimethyl ether, and the like.

In one or more embodiments, examples of the ketones include acetone, methyl ethyl ketone, methyl isobutyl ketone, cyclohexanone, and the like.

In one or more embodiments, examples of the esters include ethyl acetate, propyl acetate, butyl acetate, and the like.

In one or more embodiments, examples of the nitriles include acetonitrile, propionitrile, butyronitrile, isobutyronitrile, and the like.

In one or more embodiments, examples of the carboxylic acids include acetic acid, propionic acid, and the like.

In one or more embodiments, examples of the alcohols include methanol, ethanol, 1-propanol, 2-propanol, 1-butanol, 2-butanol, 2-methyl-1-propanol, 2-methyl-2-propanol, and the like.

In one or more embodiments, examples of the amides include formamide, N,N-dimethylformamide (DMF), N,N-dimethylacetamide, N-methyl-2-pyrrolidone, hexamethylphosphorotriamide, and the like.

In one or more embodiments, examples of the sulfoxides include dimethyl sulfoxide (DMSO), tetrahydrothiophene-1,1-dioxide, and the like.

There are no particular limitations on bases used in the reactions in steps of the methods A to E described below, as long as they do not inhibit the reactions. In one or more embodiments, examples of the bases include alkali metal carbonates, alkali metal bicarbonates, alkali metal hydroxides, alkaline earth metal hydroxides, alkali metal hydrides, alkali metal amides, alkali metal alkoxides, organic amines, and the like. Examples of the alkali metal carbonates include lithium carbonate, sodium carbonate, potassium carbonate, cesium carbonate, and the like. Examples of the alkali metal bicarbonates include lithium bicarbonate, sodium bicarbonate, potassium bicarbonate, and the like. Examples of the alkali metal hydroxides include lithium hydroxide, sodium hydroxide, potassium hydroxide, and the like. Examples of the alkaline earth metal hydroxides include calcium hydroxide, barium hydroxide, and the like. Examples of the alkali metal hydrides include lithium hydride, sodium hydride, potassium hydride, and the like. Examples of the alkali metal amides include lithium amide, sodium amide, potassium amide, and the like. Examples of the organic amines include triethylamine (TEA), tributylamine, N,N-diisopropylethylamine (DIPEA), 1-methylpiperidine, 4-methylmorpholine, 4-ethylmorpholine, pyridine, picoline, 4-dimethylaminopyridine, 4-pyrrolidinopyridine, 2,6-di-tert-butyl-4-methylpyridine, quinoline, N,N-dimethylaniline, N,N-diethylaniline, 1,5-diazabicyclo[4.3.0]-5-nonene (DBN), 1,4-diazabicyclo[2.2.2]octane (DABCO), 1,8-diazabicyclo[5.4.0]-7-undecene (DBU), imidazole, and the like.

### Method A

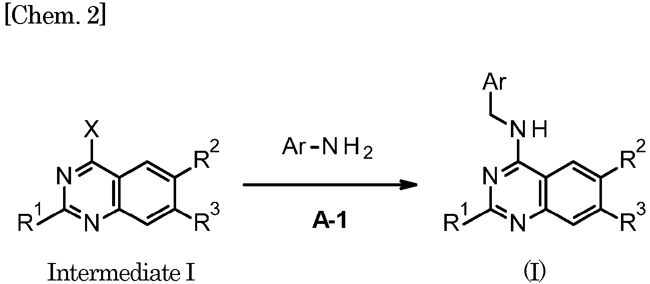

Step A-1 is a step of obtaining a compound represented by Formula (I) from an intermediate I. Step A-1 can be performed by heating in the presence of a solvent that is inert in the reaction (e.g., acetonitrile or the like), a base (e.g., triethylamine or the like), and the like. In one or more embodiments, the reaction temperature is 0°C to 100°C, and the reaction time is 30 minutes to 24 hours.

In the intermediate I and the compound represented by Formula (I), X represents a halogen atom such as a chlorine atom, and R¹, R², and R³ are as previously described regarding Formula (I). In step A-1 and in the compound represented by Formula (I), Ar is as previously described regarding Formula (I).

### Method B

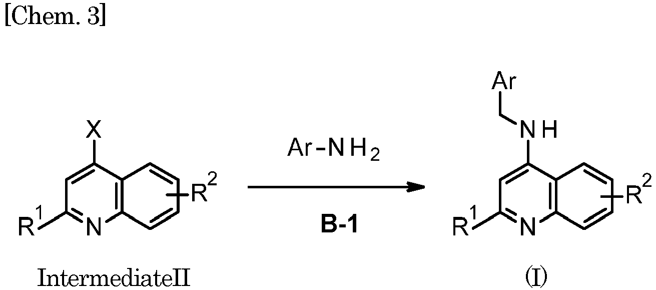

Step B-1 is a step of obtaining a compound represented by Formula (I) from an intermediate II. Step B-1 can be performed by heating in the presence of a solvent that is inert in the reaction (e.g., N-methyl-2-pyrrolidone (NMP) or the like), a base (e.g., N,N-diisopropylethylamine or the like), and the like. In one or more embodiments, the reaction temperature is 0°C to 140°C, and the reaction time is 30 minutes to 24 hours.

In the intermediate II and the compound represented by Formula (I), X represents a halogen atom such as a chlorine atom, and R¹ and R² are as previously described regarding Formula (I). In step B-1 and in the compound represented by Formula (I), Ar is as previously described regarding Formula (I).

### Method C

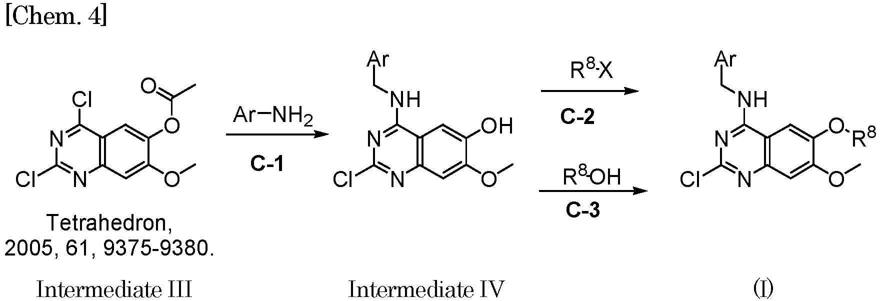

Step C-1 is a step of obtaining an intermediate IV from an intermediate III, which is reported in Tetrahedron, 2005, 61, 9375-9380. Step C-1 can be performed through a reaction in the presence of a solvent that is inert in the reaction (e.g., acetonitrile or the like) and the like. In one or more embodiments, the reaction temperature is 0°C to about room temperature, and the reaction time is 30 minutes to 24 hours.

Steps C-2 and C-3 are steps of obtaining a compound represented by Formula (I) from the intermediate IV. Steps C-2 and C-3 can be performed through a reaction in the presence of a solvent that is inert in the reaction (e.g., N,N-dimethylformamide (DMF), toluene, methanol, or the like), a base (e.g., potassium carbonate), a Mitsunobu reagent (e.g., cyanomethylenetributylphosphorane or the like), and the like. In one or more embodiments, the reaction temperature is 0°C to about 100°C, and the reaction time is 30 minutes to 24 hours.

In step C-1 and in the intermediate IV and the compound represented by Formula (I), X represents a halogen atom such as a chlorine atom. In the intermediate IV and the compound represented by Formula (I), Ar is as previously described regarding Formula (I). In steps C-2 and C-3 and in the compound represented by Formula (I), R⁸ represents R⁴ ,-(CH₂)ₜR⁵, or -(CH₂)ₜC(O)R⁵ in Formula (I) above.

### Method D

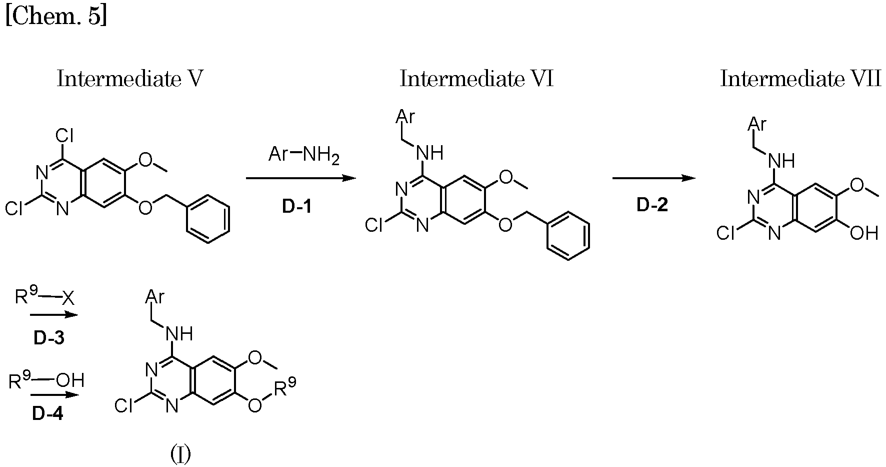

Step D-1 is a step of obtaining an intermediate VI from an intermediate V. Step D-1 can be performed through a reaction in the presence of a solvent that is inert in the reaction (e.g., tetrahydrofuran (THF) or the like), a base (e.g., triethylamine or the like), and the like. In one or more embodiments, the reaction temperature is 0°C to 100°C, and the reaction time is 30 minutes to 24 hours.

Step D-2 is a step of obtaining an intermediate VII from the intermediate VI. Step D-2 can be performed through a reaction using a transition metal catalyst (e.g., Pd/Alumina) in a solvent that is inert in the reaction (e.g., tetrahydrofuran (THF) or the like) in a hydrogen atmosphere.

Steps D-3 and D-4 are steps of obtaining a compound represented by Formula (I) from the intermediate VII. Steps D-3 and D-4 can be performed through a reaction in the presence of a solvent that is inert in the reaction (e.g., toluene, DMF, or the like), a base (e.g., potassium carbonate), and a Mitsunobu reagent (e.g., cyanomethylenetributylphosphorane or the like). In one or more embodiments, the reaction temperature is 0°C to about room temperature, and the reaction time is 30 minutes to 24 hours.

In the intermediates VI and VII and the compound represented by Formula (I), Ar is as previously described regarding Formula (I). In steps D-3 and D-4 and in the compound represented by Formula (I), R⁹ represents -(CH₂)₂NR⁶R⁷, where R⁶ and R⁷ each independently represent a hydrogen atom or a methyl group.

### Method E

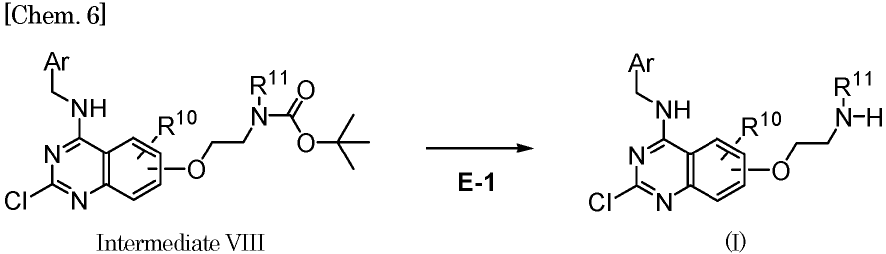

Step E-1 is a step of obtaining a compound represented by Formula (I) from an intermediate VIII. Step E-1 can be performed through a reaction in the presence of a solvent that is inert in the reaction (e.g., ethyl acetate or the like), an acid (e.g., hydrochloric acid or the like), and the like. In one or more embodiments, the reaction temperature is 0°C to room temperature, and the reaction time is 30 minutes to 24 hours.

In the intermediate VIII and the compound represented by Formula (I), Ar is as previously described regarding Formula (I), R¹⁰ is a methoxy group, and R¹¹ is a hydrogen atom or a methyl group.

In one or more embodiments, the compound of the present disclosure or pharmaceutically acceptable salt thereof has an effect capable of suppressing aberrant splicing that contributes to the onset or progression of a genetic disease. In one or more embodiments, the compound of the present disclosure or pharmaceutically acceptable salt thereof can be used to treat a genetic disease caused by aberrant splicing.

The term "genetic disease caused by aberrant splicing" as used in the present disclosure encompasses, in one or more embodiments, genetic diseases caused by aberrant splicing due to exon skipping type mutations, splice-site selection type mutations, intron retention type mutations, pseudoexon type mutations, and the like. An exon skipping type mutation refers to a splicing mutation in which a normally recognized exon is no longer recognized (skipping occurs) due to a mutation in that exon or a neighboring intron sequence. A splice-site selection mutation refers to a splicing mutation in which a plurality of 5' splice sites or 3' splice sites are generated due to a mutation in a splicing regulatory sequence in an exon region or an intron region. An intron retention type mutation refers to a splicing mutation in which recognition of an intron region is incomplete due to a mutation in an exon or intron region around a 5' splice site or a 3' splice site, inducing intron retention. A pseudoexon type mutation refers to a splicing mutation in which a sequence originally from an intron region is recognized as an exon due to a mutation.

Among exon skipping type mutations, mutation types excluding mutations at GU located at the 5' splice site +1 and +2, and AG located at the 3' splice site -1 and -2, which are essential for splicing, are expected to be targets for splicing therapeutics. In one or more embodiments, examples of the genetic diseases caused by aberrant splicing due to exon skipping type mutations include familial dysautonomia, congenital long QT syndrome, Fabry disease, and the like.

Familial dysautonomia is a genetic disease caused by a T>C single nucleotide substitution (IVS20+6T>C) at the sixth nucleotide of the intron downstream of exon 20 of the IKBKAP gene, the substitution causing exon 20 to be unrecognized, leading to exon skipping, which in turn results in decreased expression levels of the gene product IKAP and aberrant modification of tRNAs that recognize nucleotide base codes.

Congenital long QT syndrome is a genetic disease characterized by syncope and sudden death due to QT interval prolongation and polymorphic ventricular tachycardia, and is considered to be caused by genetic abnormalities in ion channel-related molecules. Regarding the sites of genetic mutations, mutations in exon regions account for only about 70% of all mutations, while the remaining about 30% are indicated to be associated with mutations in intron regions. Hereditary congenital long QT syndrome encompasses autosomal dominant Romano-Ward syndrome and autosomal recessive Jervell and Lange-Nielsen syndrome. Thirteen genotypes (LQT1 to LQT13) have been reported for Romano-Ward syndrome, and two genotypes (JLN1 and JLN2) have been reported for Jervell and Lange-Nielsen syndrome. Reported causative genes include KCNQ1 (LQT1, JLN1), KCNH2 (LQT2, JLN2), SCN5A (LQT3), ANK2 (LQT4), KCNE1 (LQT5), KCNE2 (LQT6), KCNJ2 (LQT7), CACNA1C (LQT8), CAV3 (LQT9), SCN4B (LQT10), AKAP-9 (LQT11), SNTA1 (LQT12), and KCNJ5 (LQT13). Among these, mutations in the KCNQ1, KCNH2, and SCN5A genes have been reported to occur more frequently.

KCNQ1 (α subunit), KCNH2 (α subunit), KCNE1 (β subunit), and KCNE2 (β subunit) are genes encoding the α or β subunit of a potassium channel. KCNQ1 encodes the α subunit of a voltage-dependent potassium channel, and four KCNQ1 subunit molecules assemble to form a single KCNQ1 channel.

In one or more embodiments, examples of mutations in congenital long QT syndrome include mutations in KCNQ1 (c.1032G>A), KCNH2, SCN5A, and the like.

### Pharmaceutical Composition

Another aspect of the present disclosure relates to a pharmaceutical composition containing, as an active ingredient, the compound of the present disclosure or pharmaceutically acceptable salt thereof. In one or more embodiments, the pharmaceutical composition of the present disclosure can be used to treat a genetic disease caused by aberrant splicing.

Yet another aspect of the present disclosure relates to a pharmaceutical composition for treating a genetic disease caused by aberrant splicing, the pharmaceutical composition containing, as an active ingredient, the compound of the present disclosure or pharmaceutically acceptable salt thereof.

Yet another aspect of the present disclosure relates to a pharmaceutical composition for treating a genetic disease caused by aberrant splicing, the pharmaceutical composition containing, as an active ingredient, a compound represented by Formula (I) above or a pharmaceutically acceptable salt thereof.

In one or more embodiments, the pharmaceutical composition of the present disclosure contains the compound of the present disclosure or a medicinally acceptable salt thereof and may additionally contain a pharmaceutically acceptable carrier, preservative, diluent, or excipient, or other medicinally acceptable ingredients.

In one or more embodiments, the content ratio of the compound of the present disclosure or pharmaceutically acceptable salt thereof, which is an active ingredient, in the pharmaceutical composition of the present disclosure can be appropriately determined based on the dosage form, administration method, carrier, and the like. In one or more embodiments, the pharmaceutical composition of the present disclosure can be produced according to a routine method by adding the compound of the present disclosure in a proportion of 0.01% to 100% (w/w), or 0.1% to 95% (w/w), relative to the total formulation amount.

In one or more embodiments, the pharmaceutical composition of the present disclosure can be formed in a dosage form that is suitable for the form of administration, by applying a well-known drug preparation technique. In one or more embodiments, examples of the form of administration include oral administration, parenteral administration, and the like. In one or more embodiments, examples of formulations for oral administration include those having dosage forms such as tablets, capsules, granules, powders, pills, troches, syrups, liquid medicines (e.g., solutions and suspensions), and the like. In one or more embodiments, examples of formulations for parenteral administration include injectables, aerosols, and the like. In one or more embodiments, these formulations can be produced using a well-known method with additives such as an excipient, a lubricant, a binder, a disintegrant, a stabilizing agent, a corrigent, and a diluent.

In one or more embodiments, examples of the excipient include starches such as starch, potato starch, and corn starch, lactose, crystalline cellulose, calcium hydrogen phosphate, and the like. In one or more embodiments, examples of the lubricant include ethyl cellulose, hydroxypropyl cellulose, hydroxypropyl methylcellulose, shellac, talc, carnauba wax, paraffin, and the like. In one or more embodiments, examples of the binder include polyvinylpyrrolidone, macrogol, and compounds that are similar to those given as examples of the excipient. In one or more embodiments, examples of the disintegrant include compounds that are similar to those given as examples of the excipient and chemically-modified starches and celluloses, such as croscarmellose sodium, sodium carboxymethyl starch, and crosslinked polyvinylpyrrolidone. In one or more embodiments, examples of the stabilizing agent include para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid. In one or more embodiments, examples of the corrigent include sweeteners, acidulants, flavors, and the like that are commonly used.

In one or more embodiments, to produce a liquid medicine for oral administration, ethanol, phenol, chlorocresol, purified water, distilled water, or the like can be used as a solvent. Also, a surfactant, a preservative, an isotonic agent, a pH regulator, an emulsifying agent, or the like can be used as needed. In one or more embodiments, the liquid medicine for oral administration may additionally contain a solubilizing agent, a wetting agent, a suspending agent, a sweetening agent, a flavoring agent, an aromatic agent, or a preservative.

An injectable for parenteral administration may be a sterile aqueous or non-aqueous liquid medicine, a suspension, or an emulsion. In one or more embodiments, distilled water or physiological saline may be used as an aqueous solvent for an injectable. In one or more embodiments, vegetable oil, an alcohol, or polysorbate 80 (pharmacopeia name) may be used as a non-aqueous solvent for an injectable. Examples of the vegetable oil include propylene glycol, polyethylene glycol, olive oil, and the like. Examples of the alcohol include ethanol and the like. In one or more embodiments, the injectable may additionally contain an isotonic agent, a preservative, a wetting agent, an emulsifying agent, a dispersing agent, a stabilizing agent, or a solubilizing agent. In one or more embodiments, these formulations may be sterilized by filtration through a bacteria-retaining filter, the addition of a bactericide, or irradiation with radiation. Alternatively, compositions obtained by dissolving or suspending a sterile solid composition in sterile water or a solvent for injection before use can also be used as these formulations.

The method of use of the pharmaceutical composition according to the present disclosure may vary depending on the symptoms, age, administration method, and the like. As a method of use, in one or more embodiments, the pharmaceutical composition can be intermittently or continuously administered orally, percutaneously, submucosally, subcutaneously, intramuscularly, intravascularly, intracerebrally, or intra-abdominally such that the concentration of the above compound, which is an active component, in the body is any value within a range from 100 pM to 1 mM. In a non-limiting embodiment, when the pharmaceutical composition is orally administered to a subject (an adult in the case of a human), a daily dose of 0.01 mg/kg body weight to 2000 mg/kg body weight, 0.1 mg/kg body weight to 500 mg/kg body weight, or 0.1 mg/kg body weight to 100 mg/kg body weight, in terms of the compound represented by Formula (I), may be administered to the subject at once or over multiple times depending on the symptoms. In a non-limiting embodiment, when the pharmaceutical composition is intravenously administered to a subject (an adult in the case of a human), a daily dose of 0.001 mg/kg body weight to 50 mg/kg body weight, or 0.01 mg/kg body weight to 50 mg/kg body weight may be administered to the subject at once or over multiple times depending on the symptoms.

### Treatment Method

Another aspect of the present disclosure relates to a method for treating a genetic disease caused by aberrant splicing, the method including administering the compound of the present disclosure or pharmaceutically acceptable salt thereof to a subject.

Another aspect of the present disclosure relates to use of the compound of the present disclosure or pharmaceutically acceptable salt thereof in the production of a pharmaceutical composition for use in treatment of a genetic disease caused by aberrant splicing.

The present disclosure may relate to one or more non-limiting embodiments below.
[1] A compound represented by Formula (I) below or a pharmaceutically acceptable salt thereof: 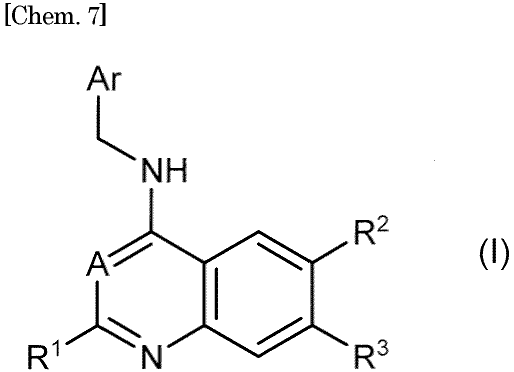 wherein, in Formula (I),
   A represents CH or N;
   R¹ represents a halogen atom;
   R² and R³ are each independently selected from the group consisting of a hydrogen atom, -OR⁴, -O(CH₂)ₜR⁵, and -O(CH₂)ₜC(O)R⁵, where at least one of R² and R³ represents -OR⁴, -O(CH₂)ₜR⁵, or -O(CH₂)ₜC(O)R⁵, and t is 1, 2, 3, or 4,
      R⁴ representing a hydrogen atom or a C₁-C₆ alkyl group, and
      R⁵ being selected from the group consisting of a C₃-C₆ cycloalkyl group, a 4- to 10-membered heterocyclyl group, -NR⁶R⁷, and -OR⁶,
      R⁶ and R⁷ each independently representing a hydrogen atom or a C₁-Cs alkyl group; and
   Ar is selected from the group consisting of a 5- to 10-membered heteroaryl group and a 6- to 12-membered aryl group, where the 5- to 10-membered heteroaryl group and the 6- to 12-membered aryl group may have one or more substituents.
[2] The compound according to clause [1] or pharmaceutically acceptable salt thereof, wherein R³ represents a methoxy group.
[3] The compound according to clause [1] or [2] or pharmaceutically acceptable salt thereof, wherein Ar represents a furanyl group, a thiophenyl group, a pyrrolyl group, a thiazolyl group, an oxazolyl group, or a pyridyl group.
[4] The compound according to any one of clauses [1] to [3] or pharmaceutically acceptable salt thereof,
   wherein R² is selected from the group consisting of a hydrogen atom, -OR⁴, - O(CH₂)ₜR⁵, and -O(CH₂)ₜC(O)R⁵,
   R⁴ representing a C₁-C₃ alkyl group,
   R⁵ representing -NR⁶R⁷, -OH, a methyl group, a cyclopropyl group, an oxetanyl group, a morpholinyl group, a 1,1-dioxo-thietanyl group, or a dioxanyl group,
   R⁶ and R⁷ each independently representing a hydrogen atom or a methyl group, and
   t being 1 or 2.
[5] The compound according to any one of clauses [1] to [4]or pharmaceutically acceptable salt thereof,
   wherein Ar represents a 2-furyl group,
   R² represents a hydrogen atom, -O(CH₂)₂CH₃, -OCH₂R⁵, or -O(CH₂)₂R⁵, where R⁵ represents -NR⁶R⁷, -OH, or an oxetanyl group, where R⁶ and R⁷ each independently represent a hydrogen atom or a methyl group, and
   R³ represents a methoxy group.
[6] A compound or a pharmaceutically acceptable salt thereof, the compound being any one compound selected from the group consisting of:
   2-chloro-N-(furan-2-ylmethyl)-6,7-dimethoxyquinazolin-4-amine,
   2-chloro-6,7-dimethoxy-N-(thiophen-2-ylmethyl)quinazolin-4-amine,
   2-chloro-6,7-dimethoxy-N-(thiophen-3-ylmethyl)quinazolin-4-amine,
   2-chloro-6,7-dimethoxy-N-(1,3-thiazol-2-ylmethyl)quinazolin-4-amine,
   2-chloro-6, 7-dimethoxy-N-(pyridin-4-ylmethyl)quinazolin-4-amine,
   2-chloro-6,7-dimethoxy-N-(1,3-oxazol-2-ylmethyl)quinazolin-4-amine,
   2-chloro-6,7-dimethoxy-N-(1H-pyrrol-3-ylmethyl)quinazolin-4-amine,
   2-chloro-N-(furan-3-ylmethyl)-6,7-dimethoxyquinazolin-4-amine,
   2-chloro-6, 7-dimethoxy-N-(pyridin-2-ylmethyl)quinazolin-4-amine,
   2-chloro-N-(furan-2-ylmethyl)-7-methoxyquinazolin-4-amine,
   2-chloro-N-(furan-2-ylmethyl)-6-methoxyquinazolin-4-amine,
   2-chloro-N-(furan-2-ylmethyl)-7-methoxyquinolin-4-amine,
   2-chloro-N-(furan-2-ylmethyl)-6-methoxyquinolin-4-amine,
   2-chloro-6-ethoxy-N-(furan-2-ylmethyl)-7-methoxyquinazolin-4-amine,
   2-chloro-6-ethoxy-N-(furan-2-ylmethyl)-7-methoxyquinazolin-4-amine,
   2-chloro-N-(furan-2-ylmethyl)-7-methoxy-6-propoxyquinazolin-4-amine,
   2-chloro-N-(furan-2-ylmethyl)-7-methoxy-6-(propan-2-yloxy)quinazolin-4-amine,
   2-chloro-N-(furan-2-ylmethyl)-7-methoxy-6-(2-methoxyethoxy)quinazolin-4-amine,
   2-chloro-6-(cyclopropylmethoxy)-N-(furan-2-ylmethyl)-7-methoxyquinazolin-4-amine,
   2-chloro-N-(furan-2-ylmethyl)-7-methoxy-6-(oxetan-3-ylmethoxy)quinazolin-4-amine,
   2-chloro-N-(furan-2-ylmethyl)-7-methoxy-6-[2-(morpholin-4-yl)ethoxy] quinazolin-4-amine,
   2-chloro-N-(furan-2-ylmethyl)-7-methoxy-6-[3-(morpholin-4-yl)propoxy]quinazolin-4-amine,
   2-chloro-6-[2-(1,1-dioxidothiethan-3-yDethoxy]-N-(furan-2-ylmethyl)-7-methoxyquinazolin-4-amine,
   6-(2-aminoethoxy)-2-chloro-N-(furan-2-ylmethyl)-7-methoxyquinazolin-4-amine,
   2-chloro-N-(furan-2-ylmethyl)-7-methoxy-6-[2-(methylamino)ethoxy]quinazolin-4-amine,
   2-chloro-6-[2-(dimethylamino)ethoxy]-N-(furan-2-ylmethyl)-7-methoxyquinazolin-4-amine,
   2-({2-chloro-4-[(furan-2-ylmethyl)amino]-7-methoxyquinazolin-6-yl}oxy)ethanol,
   2-chloro-6-(1,4-dioxan-2-ylmethoxy)-N-(furan-2-ylmethyl)-7-methoxyquinazolin-4-amine,
   3-({2-chloro-4-[(furan-2-ylmethyl)amino]-7-methoxyquinazolin-6-yl}oxy)propanoic acid,
   methyl 3-({2-chloro-4-[(furan-2-ylmethyl)amino]-7-methoxyquinazolin-6-yl}oxy)propanoate,
   methyl ({2-chloro-4-[(furan-2-ylmethyl)amino]-7-methoxyquinazolin-6-yl}oxy)acetate,
   7-(2-aminoethoxy)-2-chloro-N-(furan-2-ylmethyl)-6-methoxyquinazolin-4-amine,
   2-chloro-4-[(furan-2-ylmethyl)amino]-6-methoxyquinazolin-7-ol,
   2-chloro-N-(furan-2-ylmethyl)-6-methoxy-7-[2-(methylamino)ethoxy]quinazolin-4-amine, and
   2-chloro-7-ethoxy-N-(furan-2-ylmethyl)-6-methoxyquinazolin-4-amine.
[7] A pharmaceutical composition containing, as an active ingredient, the compound according to any one of clauses [1] to [6] or pharmaceutically acceptable salt thereof.
[8] A pharmaceutical composition for treating a genetic disease caused by aberrant splicing, the pharmaceutical composition containing, as an active ingredient, the compound according to any one of clauses [1] to [6] or pharmaceutically acceptable salt thereof.
[9] A pharmaceutical composition for treating a genetic disease caused by aberrant splicing,
   the pharmaceutical composition containing, as an active ingredient, a compound represented by Formula (I) below or a pharmaceutically acceptable salt thereof: 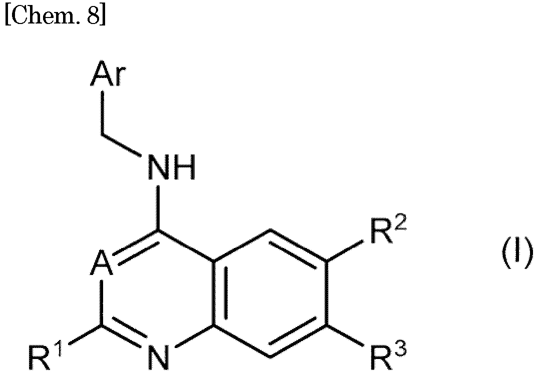 wherein, in Formula (I),
   A represents CH or N;
   R¹ represents a halogen atom;
   R² and R³ are each independently selected from the group consisting of a hydrogen atom, -OR⁴, -O(CH₂)ₜR⁵, and -O(CH₂)ₜC(O)R⁵, where at least one of R² and R³ represents -OR⁴, -O(CH₂)ₜR⁵, or -O(CH₂)ₜC(O)R⁵, and t is 1, 2, 3, or 4,
      R⁴ representing a hydrogen atom or a C₁-C₆ alkyl group, and
      R⁵ being selected from the group consisting of a C₃-C₆ cycloalkyl group, a 4- to 10-membered heterocyclyl group, -NR⁶R⁷, and -OR⁶,
      R⁶ and R⁷ each independently representing a hydrogen atom or a C₁-Cs alkyl group; and
   Ar is selected from the group consisting of a 5- to 10-membered heteroaryl group and a 6- to 12-membered aryl group, where the 5- to 10-membered heteroaryl group and the 6- to 12-membered aryl group may have one or more substituents.
[10] The pharmaceutical composition according to clause [9],
   wherein Ar represents a 2-furyl group,
   R² represents a hydrogen atom, -O(CH₂)₂CH₃, -OCH₂R⁵, or -O(CH₂)₂R⁵, where R⁵ represents -NR⁶R⁷, -OH, or an oxetanyl group, where R⁶ and R⁷ each independently represent a hydrogen atom or a methyl group, and
   R³ represents a methoxy group.
[11] The pharmaceutical composition according to any one of clause [8] to [10], wherein the genetic disease caused by aberrant splicing is familial dysautonomia or congenital long QT syndrome.
[12] A method for treating a genetic disease caused by aberrant splicing, the method including administering the compound according to any one of clauses [1] to [6] or pharmaceutically acceptable salt thereof, or the pharmaceutical composition according to any one of clauses [7] to [11], to a subject.
[13] The method according to clause [12], wherein the genetic disease caused by aberrant splicing is familial dysautonomia or congenital long QT syndrome.
[14] Use of the compound according to any one of clauses [1] to [6] or pharmaceutically acceptable salt thereof in production of a pharmaceutical composition for use in treatment of a genetic disease caused by aberrant splicing.
[15] The use according to clause [14], wherein the genetic disease caused by aberrant splicing is familial dysautonomia or congenital long QT syndrome.

### Examples

Hereinafter, the present disclosure will be described in further detail using examples. However, the scope of the present disclosure is not limited to the following examples, and these examples should not be construed in any way as limiting. Reagents, solvents, and starting materials not specifically described herein are readily available from commercial sources.

Proton nuclear magnetic resonance spectra (1H-NMR) were measured using a 400 MHz NMR spectrometer manufactured by JEOL Ltd., a 400 MHz NMR spectrometer manufactured by Varian, or a 400 MHz NMR spectrometer manufactured by Bruker. The notation of the spectral data indicates significant peaks, showing the chemical shift (expressed as relative ppm (δ) using tetramethylsilane as the reference material), the number of protons, and the signal multiplicity (expressed as follows: s: singlet; d: doublet; t: triplet; q: quartet; quint: quintet; m: multiplet; br: broad; br s: broad singlet; etc.). Also, when clearly identifiable, the spin-spin coupling constant was expressed as a J value (in Hz).

Mass spectra (MS m/z) were measured using electrospray ionization (ESI) or atmospheric pressure chemical ionization (APCI). Mass spectral data indicate maximum ionization peaks, which in most cases coincide with maximum UV absorption peaks, after passage through reversed-phase high-performance liquid chromatography columns (Agilent system; columns: Develosil Combi-RP-5, 2.0 × 50 mm, Cadenza CD-C18, 3.0 × 75mm, or ZORBAX SB-C18, 1.8 µm, 2.1 × 50 mm; and solvents: either a 0.1% formic acid-containing acetonitrile/water system or a 0.01% trifluoroacetic acid-containing acetonitrile/water system).

Silica gel column chromatography was performed using a commercially available packed column and an automated preparative purification apparatus (e.g., SP1 manufactured by Biotage, EPCLC-W-Prep 2XY manufactured by Yamazen Corporation, or Purif-α2 manufactured by Shoko Science Co., Ltd.), and only multiple solvent species used as mobile phases are described. Elution was performed under observation by thin layer chromatography (TLC). The TLC plates used were Silica gel 60 F254 or 60 NH2 F254S manufactured by Merck, NH2 Silica Gel 60 F254 plates manufactured by Wako Pure Chemical Industries, Ltd., or CHROMATOREX NH TLC manufactured by Fuji Silysia Chemical Ltd. The developing solvents used were the mobile phases used in the column chromatography. As the detection methods, a UV detector or a color reagent was used. Note that "silica gel column chromatography (NH)" in the following examples refers to silica gel whose surface is chemically modified with a functional group having an amino group (e.g., Purif-Pack (registered trademark, Shoko Scientific) EX and NH series, etc.). "Silica gel column chromatography (C18)" in the following examples refers to silica gel whose surface is chemically modified with an octadecyl group (e.g., SNAP Ultra C18 series etc.).

### (Example 1) 2-Chloro-N-(furan-2-ylmethyl)-6,7-dimethoxyquinazolin-4-amine

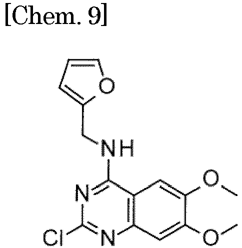

Furfurylamine (39.0 µL, 0.417 mmol) and triethylamine (67.0 µL, 0.417 mmol) were added to a suspension (1.5 mL) of 2,4-dichloro-6, 7-dimethoxyquinazoline (103 mg, 0.398 mmol) in acetonitrile, followed by stirring at 70°C for 2.5 hours. After the reaction solution was allowed to cool to room temperature, water (7 mL) was added, and an insoluble material was collected by filtration. Then, the solid was washed with a solvent mixture of acetonitrile/water (6/1), and then dried to obtain the title compound (119 mg, 94% yield) as a white solid.

### (Example 2) 2-Chloro-6,7-dimethoxy-N-(thiophen-2-ylmethyl)quinazolin-4-amine

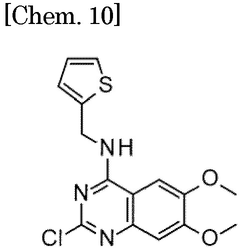

The same operation as in Example 1 was performed using 2,4-dichloro-6,7-dimethoxyquinazoline (203 mg, 0.784 mmol) and 2-thiophenemethylamine (82.4 µL, 0.823 mmol). Thus, the title compound (237 mg, 90% yield) was obtained as a pale yellow solid.

### (Example 3) 2-Chloro-6,7-dimethoxy-N-(thiophen-3-ylmethyl)quinazolin-4-amine

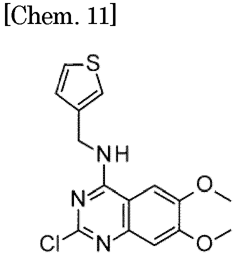

The same operation as in Example 1 was performed using 2,4-dichloro-6,7-dimethoxyquinazoline (203 mg, 0.784 mmol) and 3-thiophenemethylamine (82.4 µL, 0.823 mmol). Thus, the title compound (241 mg, 92% yield) was obtained as a pale yellow solid.

### (Example 4) 2-Chloro-6,7-dimethoxy-N-(1,3-thiazol-2-ylmethyl)quinazolin-4-amine

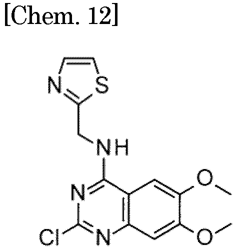

The same operation as in Example 1 was performed using 2,4-dichloro-6,7-dimethoxyquinazoline (203 mg, 0.784 mmol) and 2-(aminomethyl)thiazole (78.1 µL, 0.824 mmol). Thus, the title compound (229 mg, 87% yield) was obtained as a pale yellow solid.

### (Example 5) 2-Chloro-6,7-dimethoxy-N-(pyridin-4-ylmethyl)quinazolin-4-amine

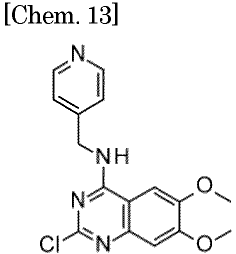

The same operation as in Example 1 was performed using 2,4-dichloro-6,7-dimethoxyquinazoline (203 mg, 0.784 mmol) and 4-aminomethylpyridine (83.2 µL, 0.823 mmol). Thus, the title compound (225 mg, 87% yield) was obtained as a brown solid.

### (Example 6) 2-Chloro-6,7-dimethoxy-N-(1,3-oxazol-2-ylmethyl)quinazolin-4-amine

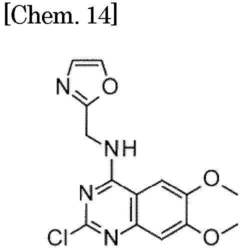

The same operation as in Example 1 was performed using 2,4-dichloro-6,7-dimethoxyquinazoline (203 mg, 0.784 mmol) and 2-aminomethyl-oxazole hydrochloride (111 mg, 0.823 mmol). Thus, the title compound (158 mg, 63% yield) was obtained as a pale brown solid.

### (Example 7) 2-Chloro-6,7-dimethoxy-N-(1H-pyrrol-3-ylmethyl) quinazolin-4-amine

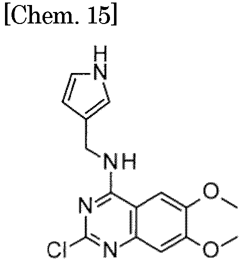

The same operation as in Example 1 was performed using 2,4-dichloro-6,7-dimethoxyquinazoline (204 mg, 0.787 mmol) and (1H-pyrrol-3-yl)methanamine (72.3 µL, 0.827 mmol). Thus, the title compound (208 mg, 83% yield) was obtained as a pale brown solid.

### (Example 8) 2-Chloro-N-(furan-3-ylmethyl)-6,7-dimethoxyquinazolin-4-amine

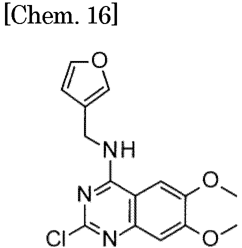

The same operation as in Example 1 was performed using 2,4-dichloro-6,7-dimethoxyquinazoline (203 mg, 0.784 mmol) and 3-(aminomethyl)furan hydrochloride (110 mg, 0.823 mmol). Thus, the title compound (220 mg, 88% yield) was obtained as a light green solid.

### (Example 9) 2-Chloro-6,7-dimethoxy-N-(pyridin-2-ylmethyl)quinazolin-4-amine

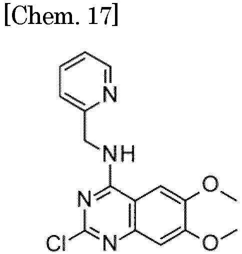

The same operation as in Example 1 was performed using 2,4-dichloro-6,7-dimethoxyquinazoline (204 mg, 0.787 mmol) and 2-picolylamine (83.6 µL, 0.827 mmol). Thus, the title compound (231 mg, 89% yield) was obtained as a pale white solid.

### (Example 10) 2-Chloro-N-(furan-2-ylmethyl)-7-methoxyquinazolin-4-amine

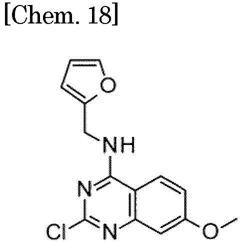

The same operation as in Example 1 was performed using 2,4-dichloro-7-methoxyquinazoline (118 mg, 0.515 mmol) and furfurylamine (48.0 µL, 0.515 mmol). Thus, the title compound (149 mg, 100% yield) was obtained as a pale white solid.

### (Example 11) 2-Chloro-N-(furan-2-ylmethyl)-6-methoxyquinazolin-4-amine

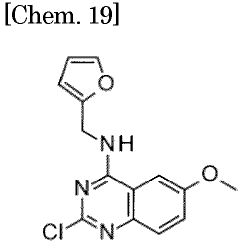

The same operation as in Example 1 was performed using 2,4-dichloro-6-methoxyquinazoline (101 mg, 0.441 mmol) and furfurylamine (43.0 µL, 0.463 mmol). Thus, the title compound (127 mg, 99% yield) was obtained as a white solid.

### (Example 12) 2-Chloro-N-(furan-2-ylmethyl)-7-methoxyquinolin-4-amine

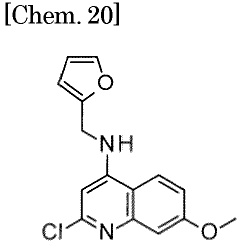

Furfurylamine (44.0 µL, 0.472 mmol) and N,N-diisopropylethylamine (149 µL, 0.858 mmol) were added to a solution (1 mL) of 2,4-dichloro-7-methoxyquinoline (97.8 mg, 0.429 mmol) in NMP, followed by stirring at 120°C for 11 hours. The reaction solution was allowed to cool to room temperature. Then, water (7 mL) was added, followed by extraction with ethyl acetate and subsequent concentration under reduced pressure. The resulting residue was purified by silica gel column chromatography (ethyl acetate/hexane = 0/100 to 20/80) to obtain the title compound (25.1 mg, 20% yield) as a yellow solid. Also, a positional isomer, 4-chloro-N-(furan-2-ylmethyl)-7-methoxyquinolin-2-amine (14.6 mg, 12% yield) was obtained as a yellow solid.

### (Example 13) 2-Chloro-N-(furan-2-ylmethyl)-6-methoxyquinolin-4-amine

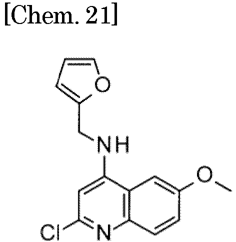

The same operation as in Example 12 was performed using furfurylamine (42.0 µL, 0.455 mmol) in a solution (1 mL) of 2,4-dichloro-6-methoxyquinoline (94.4 mg, 0.414 mmol) in NMP. Thus, the title compound (19.5 mg, 16% yield) was obtained as a yellow solid.

### (Example 14) 2-Chloro-6-ethoxy-N-(furan-2-ylmethyl)-7-methoxyquinazolin-4-amine

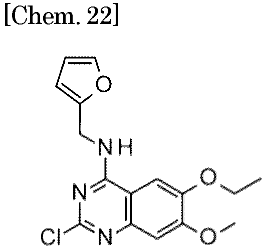

### (Example 14-1) 2-Chloro-4-[(furan-2-ylmethyl)amino]-7-methoxyquinazolin-6-ol

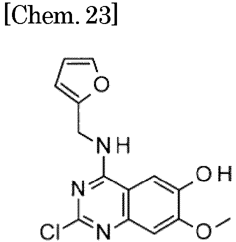

Furfurylamine (27.1 g, 279 mmol) was added to a solution (120 mL) of 2,4-dichloro-7-methoxyquinazolin-6-yl acetate (8.00 g, 27.9 mmol), reported in Tetrahedron, 2005, 61, 9375-9380, in acetonitrile, followed by stirring at room temperature for 17 hours. A 5% aqueous citric acid solution (550 mL) was added to the reaction solution, and the precipitated solid was collected by filtration, washed with water, and then air-dried. The resulting brown solid was purified by silica gel column chromatography (C18, water/methanol = 70/30 to 40/60) to obtain the title compound (8.22 g, 96% yield) as a white solid.

### (Example 14-2) 2-Chloro-6-ethoxy-N-(furan-2-ylmethyl)-7-methoxyquinazolin-4-amine

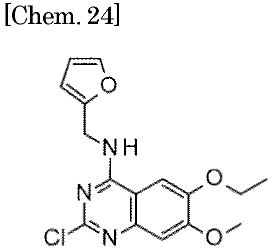

Potassium carbonate (136 mg, 0.981 mmol) and ethyl iodide (47.1 µL, 0.589 mmol) were added to a solution (3 mL) of 2-chloro-4-[(furan-2-ylmethyl)amino]-7-methoxyquinazolin-6-ol (150 mg, 0.491 mmol) obtained in Example 14-1 in DMF, followed by stirring at room temperature for 4 hours. Water (30 mL) was added to the reaction solution, and the mixture was stirred for 20 minutes. Then, the precipitated solid was collected by filtration, washed with water, and then dried to obtain the title compound (141 mg, 86% yield) as a yellowish white solid.

### (Example 15) 2-Chloro-N-(furan-2-ylmethyl)-7-methoxy-6-propoxyquinazolin-4-amine

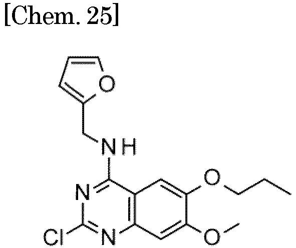

Potassium carbonate (136 mg, 0.981 mmol) and propyl iodide (57.2 µL, 0.589 mmol) were added to a solution (3 mL) of 2-chloro-4-[(furan-2-ylmethyl)amino]-7-methoxyquinazolin-6-ol (150 mg, 0.491 mmol) obtained in Example 14-1 in DMF, followed by stirring at room temperature for 4 hours. Water (30 mL) was added to the reaction solution, and the mixture was stirred for 40 minutes. Then, the precipitated solid was collected by filtration, washed with water, and then dried to obtain the title compound (126 mg, 74% yield) as a yellowish white solid.

### (Example 16) 2-Chloro-N-(furan-2-ylmethyl)-7-methoxy-6-(propan-2-yloxy)quinazolin-4-amine

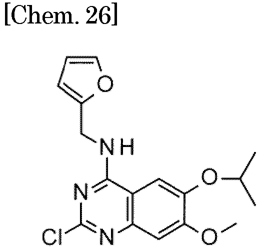

Potassium carbonate (181 mg, 1.31 mmol) and 2-iodopropane (78.1 µL, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 22 hours. Water (30 mL) was added to the reaction solution, and the mixture was stirred. Then, the precipitated solid was collected by filtration, washed with water, and then dried to obtain the title compound (193 mg, 85% yield) as a white solid.

### (Example 17) 2-Chloro-N-(furan-2-ylmethyl)-7-methoxy-6-(2-methoxyethoxy)quinazolin-4-amine

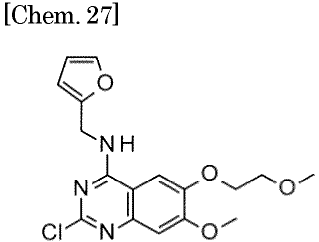

Potassium carbonate (181 mg, 1.31 mmol) and 2-bromoethyl methyl ether (74.4 µL, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 21 hours. Water (30 mL) was added to the reaction solution, and the mixture was stirred. Then, the precipitated solid was collected by filtration, washed with water, and then dried to obtain the title compound (211 mg, 89% yield) as a white solid.

### (Example 18) 2-Chloro-6-(cyclopropylmethoxy)-N-(furan-2-ylmethyl)-7-methoxyquinazolin-4-amine

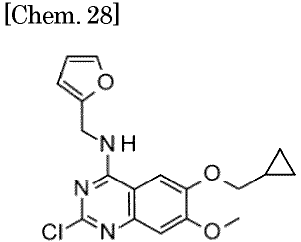

Potassium carbonate (181 mg, 1.31 mmol) and iodomethyl cyclopropane (73.2 µL, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 23 hours. Water (30 mL) was added to the reaction solution, and the mixture was stirred. Then, the precipitated solid was collected by filtration, washed with water, and then dried to obtain the title compound (193 mg, 82% yield) as a white solid.

### (Example 19) 2-Chloro-N-(furan-2-ylmethyl)-7-methoxy-6-(oxetan-3-ylmethoxy)quinazolin-4-amine

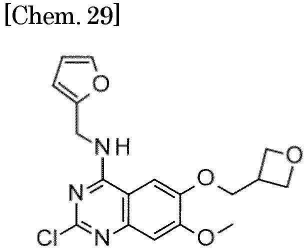

Potassium carbonate (181 mg, 1.31 mmol) and 3-bromomethyl oxetane (75.6 µL, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 24 hours. Water (30 mL) was added to the reaction solution, and the mixture was stirred. Then, the precipitated solid was collected by filtration, washed with water, and then dried to obtain a crudely purified product. The crudely purified product was purified by silica gel column chromatography (C18, water/methanol = 70/30 to 30/70) to obtain the title compound (163 mg, 66% yield) as a white solid.

### (Example 20) 2-Chloro-N-(furan-2-ylmethyl)-7-methoxy-6-[2-(morpholin-4-yl)ethoxy] quinazolin-4-amine

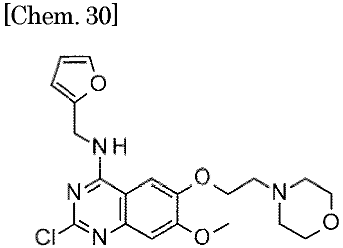

4-(2-Hydroxyethyl)morpholine (160 µL, 1.31 mmol) and cyanomethylenetributylphosphorane (344 µL, 1.31 mmol) were added to a solution (5 mL) of 2-chloro-4-[(furan-2-ylmethyl)amino]-7-methoxyquinazolin-6-ol (200 mg, 0.654 mmol) obtained in Example 14-1 in toluene, followed by stirring at 80°C for 3 hours. 4-(2-Hydroxyethyl)morpholine (40.0 µL, 0.327 mmol) and cyanomethylenetributylphosphorane (85.9 µL, 0.327 mmol) were added to the reaction solution, and the mixture was further stirred at 80°C for 17 hours. The reaction solution was concentrated, and the resulting residue was purified by silica gel column chromatography (C18, 0.1% aqueous trifluoroacetic acid/methanol = 70/30 to 40/60). Fractions containing the target product were collected, neutralized with a saturated aqueous solution of sodium bicarbonate, and then concentrated. The precipitated solid was collected by filtration and dried to obtain the title compound (73.6 mg, 27% yield) as a grayish white solid.

### (Example 21) 2-Chloro-N-(furan-2-ylmethyl)-7-methoxy-6-[3-(morpholin-4-yl)propoxy]quinazolin-4-amine

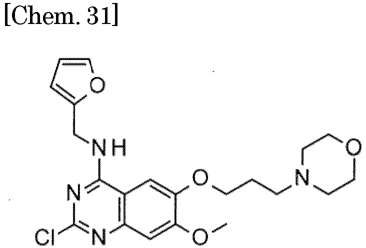

4-(3-Hydroxypropyl)morpholine (182 µL, 1.31 mmol) and cyanomethylenetributylphosphorane (344 µL, 1.31 mmol) were added to a solution (5 mL) of 2-chloro-4-[(furan-2-ylmethyl)amino]-7-methoxyquinazolin-6-ol (200 mg, 0.654 mmol) obtained in Example 14-1 in toluene, followed by stirring at 80°C for 3 hours. 4-(3-Hydroxypropyl)morpholine (45.4 µL, 0.327 mmol) and cyanomethylenetributylphosphorane (85.9 µL, 0.327 mmol) were added to the reaction solution, and the mixture was further stirred at 80°C for 17 hours. The reaction solution was concentrated, and the resulting residue was purified by silica gel column chromatography (C18, 0.1% aqueous trifluoroacetic acid/methanol = 70/30 to 40/60). Fractions containing the target product were collected, neutralized with a saturated aqueous solution of sodium bicarbonate, and then concentrated, followed by extraction with ethyl acetate. Subsequently, the organic layer was washed with a saturated saline solution, dried over anhydrous sodium sulfate, and then concentrated under reduced pressure. The resulting residue was purified by silica gel column chromatography (NH, ethyl acetate) to obtain the title compound (76.2 mg, 27% yield) as a grayish white solid.

### (Example 22) 2-Chloro-6-[2-(1,1-dioxidothiethan-3-yl)ethoxy]-N-(furan-2-ylmethyl)-7-methoxyquinazolin-4-amine

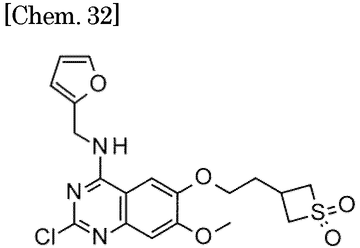

Potassium carbonate (136 mg, 0.981 mmol) and 3-(2-bromoethyl)thietane 1,1-dioxide (126 mg, 0.591 mmol) were added to a solution (3 mL) of 2-chloro-4-[(furan-2-ylmethyl)amino]-7-methoxyquinazolin-6-ol (150 mg, 0.491 mmol) obtained in Example 14-1 in DMF, followed by stirring at room temperature for 24 hours. Water (30 mL) was added to the reaction solution, and the mixture was stirred for 30 minutes. Then, the precipitated solid was collected by filtration, washed with water, and then dried to obtain the title compound (185 mg, 86% yield) as a pale yellow solid.

### (Example 23) 6-(2-Aminoethoxy)-2-chloro-N-(furan-2-ylmethyl)-7-methoxyquinazolin-4-amine

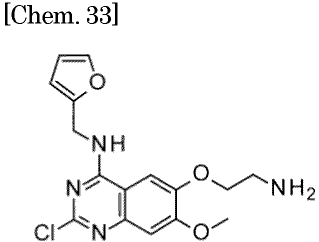

Potassium carbonate (181 mg, 1.31 mmol) and 2-(tert-butoxycarbonylamino)ethyl bromide (176 mg, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 19 hours. Water (30 mL) was added to the reaction solution, and the mixture was stirred, followed by extraction with ethyl acetate. Subsequently, the organic layer was washed with a saturated saline solution, dried over anhydrous sodium sulfate, and then concentrated under reduced pressure. 4N Hydrochloric acid-ethyl acetate (8 mL) was added to a solution (2 mL) of the resulting brown oily substance in ethyl acetate, and the mixture was stirred at room temperature for 3 hours. The reaction solution was concentrated under reduced pressure, and water/methanol (1/1,10 mL) was added to the resulting pale yellow solid to form a solution, which was then neutralized with a saturated aqueous solution of sodium bicarbonate. After extraction with ethyl acetate, the organic layer was washed with a saturated saline solution, dried over anhydrous sodium sulfate, and then concentrated under reduced pressure. The resulting residue was purified by silica gel column chromatography (C18, water/acetonitrile = 80/20 to 0/100) and then lyophilized to obtain the title compound (107 mg, 47% yield) as a white solid.

### (Example 24) 2-Chloro-N-(furan-2-ylmethyl)-7-methoxy-6-[2-(methylamino)ethoxy]quinazolin-4-amine

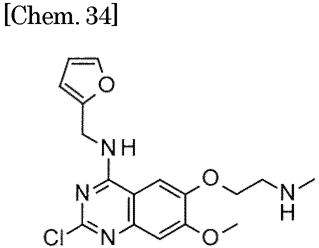

tert-Butyl(2-hydroxyethyl)(methyl)carbamate (172 mg, 0.982 mmol) and cyanomethylenetributylphosphorane (515 µL, 1.96 mmol) were added to a solution (5 mL) of 2-chloro-4-[(furan-2-ylmethyl)amino]-7-methoxyquinazolin-6-ol (200 mg, 0.654 mmol) obtained in Example 14-1 in toluene, followed by stirring at 80°C for 2.5 hours. Cyanomethylenetributylphosphorane (515 µL, 1.96 mmol) was added to the reaction solution, and the mixture was further stirred at 80°C for 1 hour. Then, the reaction solution was concentrated under reduced pressure. The resulting residue was purified by silica gel column chromatography (C18, water/methanol = 70/30 to 20/80) to obtain 247 mg of a pale yellow oily substance. 4N Hydrochloric acid-ethyl acetate (5 mL) was added to a solution (5 mL) of the obtained pale yellow oily substance in methanol, and the mixture was stirred at room temperature for 18 hours. The reaction solution was concentrated under reduced pressure, and a saturated aqueous solution of sodium bicarbonate was added to the resulting white solid. The mixture was then extracted with ethyl acetate containing 10% THF, followed by drying over anhydrous sodium sulfate and subsequent concentration under reduced pressure. The resulting residue was purified by silica gel column chromatography (C18, water/methanol = 70/30 to 0/100), and the resulting crudely purified product was again purified by silica gel column chromatography (C18, 0.1% aqueous trifluoroacetic acid/methanol = 80/20 to 40/60). Fractions containing the target product were concentrated, neutralized with a saturated aqueous solution of sodium bicarbonate, and extracted with ethyl acetate, followed by washing with a saturated saline solution, drying over anhydrous sodium sulfate, and subsequent concentration under reduced pressure. Water was added to a solution of the resulting residue in methanol, and the precipitated solid was collected by filtration, washed with water, and then dried to obtain the title compound (69.0 mg, 29% yield) as a slightly reddish white solid.

### (Example 25) 2-Chloro-6-[2-(dimethylamino)ethoxy]-N-(furan-2-ylmethyl)-7-methoxyquinazolin-4-amine

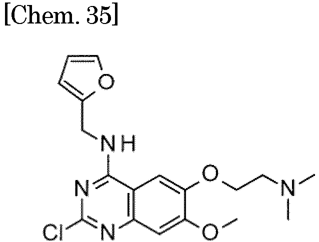

Potassium carbonate (272 mg, 1.97 mmol) and 2-bromo-N,N-dimethylethylamine hydrobromide (183 mg, 0.786 mmol) were added to a solution (4 mL) of 2-chloro-4-[(furan-2-ylmethyl)amino]-7-methoxyquinazolin-6-ol (200 mg, 0.654 mmol) obtained in Example 14-1 in THF, followed by stirring at room temperature for 3 hours. Potassium carbonate (91.0 mg, 0.658 mmol) and 2-bromo-N,N-dimethylethylamine hydrobromide (61.0 mg, 0.262 mmol) were added to the reaction solution, and the mixture was further stirred at room temperature for 3 hours. Water was added to the reaction solution, followed by extraction with ethyl acetate. Then, the organic layer was washed with a saturated saline solution, dried over anhydrous sodium sulfate, and then concentrated under reduced pressure. The resulting residue was purified by silica gel column chromatography (NH, ethyl acetate) and lyophilized to obtain the title compound (144 mg, 59% yield) as a white solid.

### (Example 26) 2-({2-Chloro-4-[(furan-2-ylmethyl)amino]-7-methoxyquinazolin-6-yl}oxy)ethanol

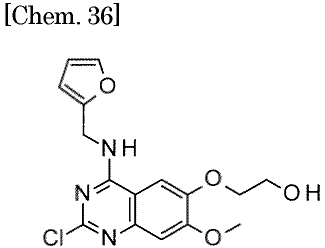

Potassium carbonate (181 mg, 1.31 mmol) and 2-bromoethanol (55.6 µL, 0.785 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 3 hours. 2-Bromoethanol (23.1 µL, 0.326 mmol) was added to the reaction solution, and then, potassium carbonate (181 mg, 1.31 mmol) and 2-bromoethanol (55.6 µL, 0.785 mmol) were added after 17 hours and 40 hours. The mixture was stirred for a total of 44 hours. Water (100 mL) was added to the reaction solution, and the precipitated solid was collected by filtration. The resulting solid was purified by silica gel column chromatography (C18, water/methanol = 70/30 to 30/70) to obtain the title compound (133 mg, 58% yield) as a white solid.

### (Example 27) 2-Chloro-6-(1,4-dioxan-2-ylmethoxy)-N-(furan-2-ylmethyl)-7-methoxyquinazolin-4-amine

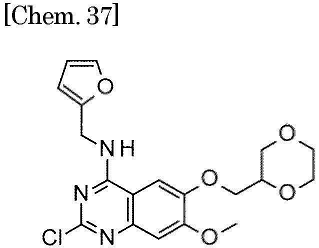

(1,4-Dioxan-2-yl)methanol (69.3 µL, 0.655 mmol) and cyanomethylenetributylphosphorane (172 µL, 0.654 mmol) were added to a solution (3 mL) of 2-chloro-4-[(furan-2-ylmethyl)amino]-7-methoxyquinazolin-6-ol (100 mg, 0.327 mmol) obtained in Example 14-1 in toluene, followed by stirring at 80°C for 4 hours. Cyanomethylenetributylphosphorane (172 µL, 0.654 mmol) was added to the reaction solution, and the mixture was stirred at 80°C for an additional 3 hours. The reaction solution was allowed to cool to room temperature and then concentrated under reduced pressure. The resulting residue was crudely purified by silica gel column chromatography (NH, ethyl acetate/hexane = 50/50), then purified by silica gel column chromatography (C18, water/methanol = 70/30 to 30/70) and silica gel column chromatography (ethyl acetate/hexane = 25/75 to 50/50), and again purified by silica gel column chromatography (C18, water/methanol = 70/30 to 30/70) to obtain the title compound (80.6 mg, 61% yield) as a pale white solid.

### (Example 28) 3-({2-Chloro-4-[(furan-2-ylmethyl)amino]-7-methoxyquinazolin-6-yl}oxy)propanoic acid

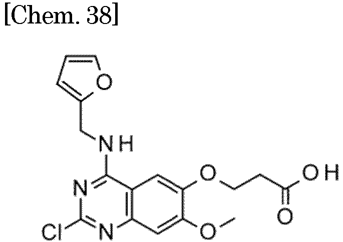

Potassium carbonate (814 mg, 5.89 mmol) and 3-bromopropionic acid (360 mg, 2.36 mmol) were added to a solution (8 mL) of 2-chloro-4-[(furan-2-ylmethyl)amino]-7-methoxyquinazolin-6-ol (600 mg, 1.96 mmol) obtained in Example 14-1 in DMF, followed by stirring at room temperature for 16 hours. Potassium carbonate (271 mg, 1.96 mmol) and 3-bromopropionic acid (300 mg, 1.96 mmol) were added to the reaction solution, and the mixture was stirred for an additional 6 hours. Water (20 mL) and methanol (20 mL) were added to the reaction solution, followed by stirring for 16 hours. Then, the reaction solution was concentrated under reduced pressure. Citric acid was added to the resulting residue to adjust the pH to 4 to 5, followed by extraction with ethyl acetate. The organic layer was washed with water and a saturated saline solution, then dried over anhydrous sodium sulfate, and concentrated under reduced pressure. The resulting residue was purified by silica gel column chromatography (ethyl acetate/hexane = 50/50 to 100/0) to obtain the title compound (213 mg, 29% yield) as a white solid.

### (Example 29) Methyl 3-({2-chloro-4-[(furan-2-ylmethyl)amino]-7-methoxyquinazolin-6-yl}oxy)propanoate

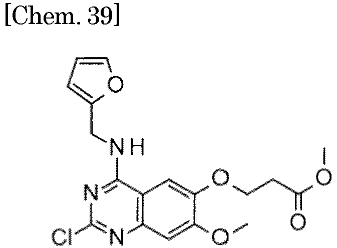

3-({2-Chloro-4-[(furan-2-ylmethyl)amino]-7-methoxyquinazolin-6-yl}oxy)propanoic acid (100 mg, 0.265 mmol) obtained in Example 28 was added to a solution (10 mL) of thionyl chloride (28.3 µL, 0.397 mmol) in methanol cooled to -18°C, and the mixture was stirred for 10 minutes, followed by stirring at 50°C for 1 hour. The reaction solution was allowed to cool to room temperature and then concentrated under reduced pressure. Methanol (3 mL) was added to the resulting residue, and a saturated aqueous solution of sodium bicarbonate was then added. The precipitated pale yellow solid was collected by filtration. The obtained crudely purified product was purified by silica gel column chromatography (C18, water/methanol = 70/30 to 30/70) to obtain the title compound (73.2 mg, 71% yield) as a white solid.

### (Example 30) Methyl ({2-chloro-4-[(furan-2-ylmethyl)amino]-7-methoxyquinazolin-6-yl}oxy)acetate

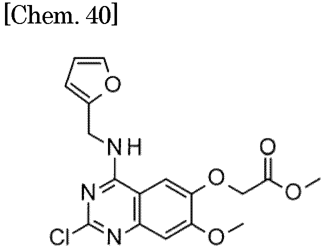

Potassium carbonate (181 mg, 1.31 mmol) and methyl bromoacetate (72.3 µL, 0.786 mmol) were added to a solution (3 mL) of 2-chloro-4-[(furan-2-ylmethyl)amino]-7-methoxyquinazolin-6-ol (200 mg, 0.654 mmol) obtained in Example 14-1 in DMF, followed by stirring at room temperature for 4 hours. A 5% aqueous citric acid (30 mL) was added to the reaction solution, and the precipitated solid was collected by filtration. The resulting crudely purified product was purified by silica gel column chromatography (C18, 0.1% aqueous trifluoroacetic acid/methanol = 70/30 to 30/70). Fractions containing the target product were neutralized with a saturated aqueous solution of sodium bicarbonate and then concentrated. The precipitated solid was collected by filtration, washed with water, and then dried to obtain the title compound (161 mg, 65% yield) as a white solid.

### (Example 31) 7-(2-Aminoethoxy)-2-chloro-N-(furan-2-ylmethyl)-6-methoxyquinazolin-4-amine

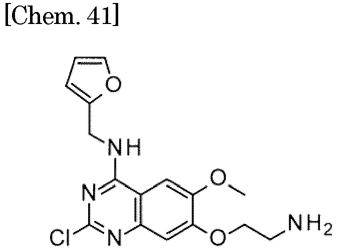

### (Example 31-1) 2-Chloro-4-[(furan-2-ylmethyl)amino]-6-methoxyquinazolin-7-ol

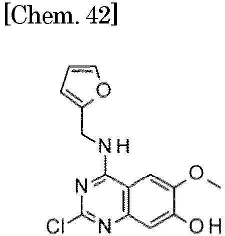

Furfurylamine (2.96 g, 30.5 mmol) and triethylamine (3.08 g, 30.4 mmol) were added to a solution (140 mL) of 7-(benzyloxy)-2,4-dichloro-6-methoxyquinazoline (8.50 g, 25.4 mmol) in THF, followed by stirring at 60°C for 6 hours. Water (280 mL) was added to the reaction solution, and the organic solvent was concentrated under reduced pressure. The precipitated solid was collected by filtration and dried to obtain 7-(benzyloxy)-2-chloro-N-(furan-2-ylmethyl)-6-methoxyquinazolin-4-amine (9.94 g, 99% yield) as a greenish white solid. 10% Pd/Alumina (2.46 g) was added to a solution (200 mL) of the obtained 7-(benzyloxy)-2-chloro-N-(furan-2-ylmethyl)-6-methoxyquinazolin-4-amine (9.84 g, 24.9 mmol) in THF at 0°C, and the mixture was stirred for 15 hours in a hydrogen atmosphere. The reaction solution was filtered through Celite. The solid was washed with methanol and then concentrated under reduced pressure. The resulting residue was purified by silica gel column chromatography (ethyl acetate/hexane = 1/2 to ethyl acetate/hexane/THF = 4/5/1), and the resulting crudely purified product was then purified by silica gel column chromatography (C18, water/methanol = 70/30 to 35/65) to obtain the title compound (5.17 g, 68% yield) as a pale yellow solid.

### (Example 31-2) 7-(2-Aminoethoxy)-2-chloro-N-(furan-2-ylmethyl)-6-methoxyquinazolin -4-amine

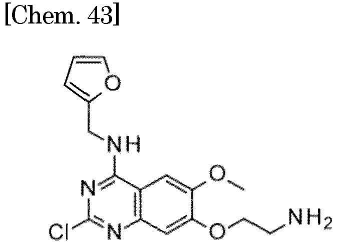

Potassium carbonate (181 mg, 1.31 mmol) and 2-(tert-butoxycarbonylamino)ethyl bromide (176 mg, 0.785 mmol) were added to a solution (3 mL) of 2-chloro-4-[(furan-2-ylmethyl)amino]-6-methoxyquinazolin-7-ol (200 mg, 0.654 mmol) obtained in Example 31-1 in DMF, followed by stirring at room temperature for 24 hours. 5% Aqueous citric acid (30 mL) was added to the reaction solution, followed by extraction with ethyl acetate. Then, the organic layer was washed with a saturated saline solution, dried over anhydrous sodium sulfate, and then concentrated under reduced pressure. The resulting residue was purified by silica gel column chromatography (C18, water/methanol = 70/30 to 30/70) to obtain a yellow oily substance. 4N Hydrochloric acid-ethyl acetate (8 mL) was added to a solution (2 mL) of the obtained yellow oily substance in ethyl acetate, and the mixture was stirred at room temperature for 17 hours. The reaction solution was concentrated under reduced pressure, and the resulting residue was purified by silica gel column chromatography (C18, 0.1% aqueous trifluoroacetic acid/methanol = 70/30 to 30/70). Fractions containing the target product were concentrated under reduced pressure. A saturated aqueous solution of sodium bicarbonate was added to the resulting residue, followed by extraction with ethyl acetate. The organic layer was washed with a saturated saline solution, dried over anhydrous sodium sulfate, and then concentrated under reduced pressure. The resulting residue was dissolved in a solvent mixture of acetonitrile/water, and the solution was lyophilized to obtain the title compound (155 mg, 68% yield) as a white solid.

### (Example 32) 2-Chloro-N-(furan-2-ylmethyl)-6-methoxy-7-[2-(methylamino)ethoxy]quinazolin-4-amine

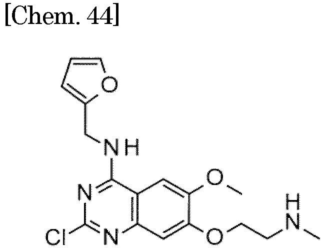

tert-Butyl(2-hydroxyethyl)(methyl)carbonate (172 mg, 0.982 mmol) and cyanomethylenetributylphosphorane (515 µL, 1.96 mmol) were added to a solution (5 mL) of 2-chloro-4-[(furan-2-ylmethyl)amino]-6-methoxyquinazolin-7-ol (200 mg, 0.654 mmol) obtained in Example 31-1 in toluene, followed by stirring at 80°C for 2 hours. The reaction solution was allowed to cool to room temperature and then concentrated under reduced pressure. The resulting residue was purified by silica gel column chromatography (C18, water/methanol = 70/30 to 20/80) to obtain a pale yellow solid. 4N Hydrochloric acid-ethyl acetate (15 mL) was added to a solution (15 mL) of the obtained solid in methanol, and the mixture was stirred at room temperature for 15 hours. The reaction solution was concentrated under reduced pressure, and the resulting residue was purified by silica gel column chromatography (C18, 0.1% aqueous trifluoroacetic acid/methanol = 80/20 to 50/50). Fractions containing the target product were concentrated under reduced pressure and then lyophilized. Water was added to a solution of the resulting pale yellow solid to form an aqueous solution, and the aqueous solution was neutralized with a saturated aqueous solution of sodium bicarbonate. The precipitated solid was collected by filtration and then dried to obtain the title compound (164 mg, 69% yield) as a pale white solid.

### (Example 33) 2-Chloro-7-ethoxy-N-(furan-2-ylmethyl)-6-methoxyquinazolin-4-amine

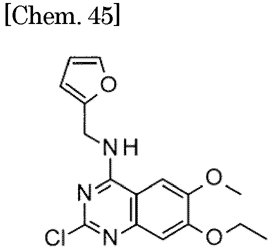

Potassium carbonate (136 mg, 0.984 mmol) and ethyl iodide (47.1 µL, 0.589 mmol) were added to a solution (3 mL) of 2-chloro-4-[(furan-2-ylmethyl)amino]-6-methoxyquinazolin-7-ol (150 mg, 0.491 mmol) obtained in Example 31-1 in DMF, followed by stirring at room temperature for 5 hours. Water (30 mL) was added to the reaction solution. The precipitated solid was collected by filtration, washed with water, and then dried to obtain the title compound (156 mg, 95% yield) as a pale white solid.

Structural formulae and physical scientific data of the compounds described in Examples 1 to 33 are shown below.

**[Table 1]**

| Example | Structural formulae | Data |
|---|---|---|
| 1 | 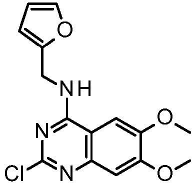 | 1H-NMR (DMSO-D6) δ: 3.87 (3H, s), 3.89 (3H, s), 4.72 (2H, d, J = 5.5 Hz), 6.37 (1H, d, J = 3.6 Hz), 6.42-6.44 (1H, m), 7.10 (1H, s), 7.62 (1H, d, J = 1.8 Hz), 7.67 (1H, s), 8.81 (1H, t, J = 5.5 Hz). |
| | | MS (m/z): 320 (M+H)⁺. |
| 2 | 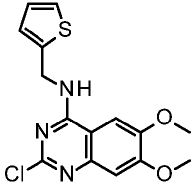 | 1H-NMR (DMSO-D6) δ: 3.87 (3H, s), 3.89 (3H, s), 4.88 (2H, d, J = 5.6 Hz), 6.98 (1H, dd, J = 5.2, 3.6 Hz), 7.10-7.12 (2H, m), 7.40 (1H, dd, J = 5.2, 1.2 Hz), 7.63 (1H, s), 8.97 (1H, t, J = 5.6 Hz). |
| | | MS (m/z): 336 (M+H)⁺. |
| 3 | 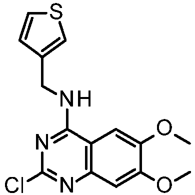 | 1H-NMR (DMSO-D6) δ: 3.87 (3H, s), 3.89 (3H, s), 4.72 (2H, d, J = 5.6 Hz), 7.09 (1H, s), 7.15 (1H, dd, J = 4.8, 0.8 Hz), 7.40 (1H, br s), 7.51 (1H, dd, J = 4.8, 2.8 Hz), 7.67 (1H, s), 8.83 (1H, t, J = 5.6 Hz). |
| | | MS (m/z): 336 (M+H)⁺. |
| 4 | 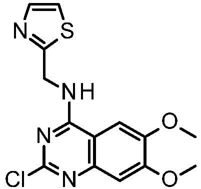 | 1H-NMR (DMSO-D6) δ: 3.88 (3H, s), 3.91 (3H, s), 5.01 (2H, d, J = 5.6 Hz), 7.13 (1H, s), 7.64 (1H, d, J = 3.2 Hz), 7.68 (1H, s), 7.76 (1H, d, J = 3.2 Hz), 9.22 (1H, t, J = 5.6 Hz). |
| | | MS (m/z): 337 (M+H)⁺. |
| 5 | 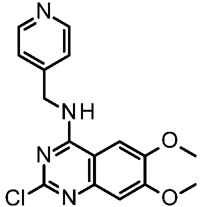 | 1H-NMR (DMSO-D6) δ: 3.89 (3H, s), 3.90 (3H, s), 4.77 (2H, d, J = 5.6 Hz), 7.12 (1H, s), 7.35 (2H, d, J = 5.6 Hz), 7.70 (1H, s), 8.52 (2H, d, J = 5.6 Hz), 8.96 (1H, t, J = 5.6 Hz). |
| | | MS (m/z): 331 (M+H)⁺. |
| 6 | 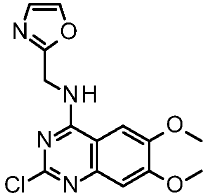 | 1H-NMR (DMSO-D6) δ: 3.89 (3H, s), 3.90 (3H, s), 4.85 (2H, d, J = 5.6 Hz), 7.12 (1H, s), 7.18 (1H, s), 7.68 (1H, s), 8.07 (1H, s), 8.90 (1H, t, J = 5.6 Hz). |
| | | MS (m/z): 321 (M+H)⁺. |
| 7 | 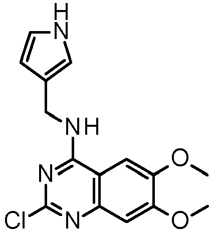 | 1H-NMR (DMSO-D6) δ: 3.85 (3H, s), 3.88 (3H, s), 4.53 (2H, d, J = 5.2 Hz), 6.08 (1H, br s), 6.69 (1H, br s), 6.77 (1H, s), 7.06 (1H, s), 7.68 (1H, s), 8.58 (1H, t, J = 5.2 Hz), 10.65 (1H, br s). |
| | | MS (m/z): 319 (M+H)⁺. |
| 8 | 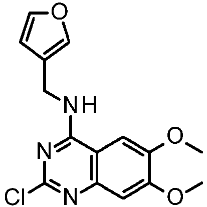 | 1H-NMR (DMSO-D6) δ: 3.87 (3H, s), 3.89 (3H, s), 4.54 (2H, d, J = 5.6 Hz), 6.53 (1H, br s), 7.09 (1H, s), 7.62-7.67 (3H, m), 8.69 (1H, t, J = 5.6 Hz). |
| | | MS (m/z): 320 (M+H)⁺. |
| 9 | 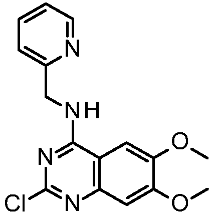 | 1H-NMR (DMSO-D6) δ: 3.89 (3H, s), 3.90 (3H, s), 4.82 (2H, d, J = 5.6 Hz), 7.11 (1H, s), 7.28-7.31 (1H, m), 7.37 (1H, d, J = 7.6 Hz), 7.73-7.79 (2H, m), 8.54 (1H, d, J = 4.4 Hz), 9.00 (1H, t, J = 5.6 Hz). |
| | | MS (m/z): 331 (M+H)⁺. |
| 10 | 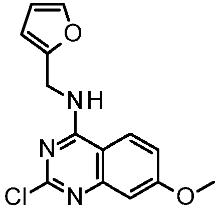 | 1H-NMR (DMSO-D6) δ: 3.88 (3H, s), 4.71 (2H, d, J = 5.5 Hz), 6.35 (1H, d, J = 3.1 Hz), 6.41-6.43 (1H, m), 7.08 (1H, d, J = 2.5 Hz), 7.15 (1H, dd, J = 9.2, 2.5 Hz), 7.61 (1H, d, J = 1.8 Hz), 8.20 (1H, d, J = 9.2 Hz), 9.03 (1H, t, J = 5.5 Hz). |
| | | MS (m/z): 290 (M+H)⁺. |
| 11 | 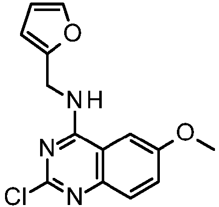 | 1H-NMR (DMSO-D6) δ: 3.87 (3H, s), 4.74 (2H, d, J = 5.5 Hz), 6.39 (1H, d, J = 3.6 Hz), 6.42-6.44 (1H, m), 7.44 (1H, dd, J = 9.1, 2.4 Hz), 7.58-7.63 (2H, m), 7.73 (1H, d, J = 2.4 Hz), 9.04 (1H, t, J = 5.5 Hz). |
| | | MS (m/z): 290 (M+H)⁺. |
| 12 | 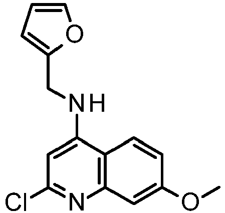 | 1H-NMR (DMSO-D6) δ: 3.86 (3H, s), 4.52 (2H, d, J = 6.1 Hz), 6.39 (1H, d, J = 2.5 Hz), 6.41 (1H, dd, J = 3.1, 1.8 Hz), 6.45 (1H, s), 7.09-7.12 (2H, m), 7.61 (1H, br d, J = 1.8 Hz), 8.01 (1H, t, J = 5.8 Hz), 8.14 (1H, d, J = 8.6 Hz). |
| | | MS (m/z): 289 (M+H)⁺. |
| 13 | 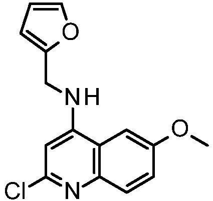 | 1H-NMR (DMSO-D6) δ: 3.88 (3H, s), 4.56 (2H, d, J = 5.5 Hz), 6.42 (2H, d, J = 1.2 Hz), 6.52 (1H, s), 7.31 (1H, dd, J = 9.2, 2.5 Hz), 7.62-7.64 (3H, m), 7.94 (1H, t, J = 5.8 Hz). |
| | | MS (m/z): 289 (M+H)⁺. |
| 14-1 | 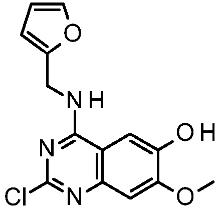 | 1H-NMR (DMSO-D6) δ: 3.92 (3H, s), 4.67 (2H, d, J = 5.6 Hz), 6.32 (1H, dd, J = 3.2, 0.8 Hz), 6.41 (1H, dd, J = 3.2, 2.0 Hz), 7.09 (1H, s), 7.52 (1H, s), 7.59 (1H, dd, J = 2.0, 0.8 Hz), 8.68 (1H, t, J = 5.6 Hz), 9.64 (1H, br s). |
| 14-2 | 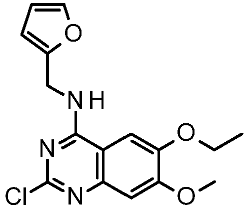 | 1H-NMR (DMSO-D6) δ: 1.40 (3H, t, J = 6.8 Hz), 3.89 (3H, s), 4.11 (2H, q, J = 6.8 Hz), 4.72 (2H, d, J = 5.2 Hz), 6.37 (1H, br s), 6.43 (1H, dd, J = 3.2, 2.0 Hz), 7.09 (1H, s), 7.62 (1H, br s), 7.66 (1H, s), 8.78 (1H, t, J = 5.2 Hz). |
| | | MS (m/z): 334 (M+H)⁺. |
| 15 | 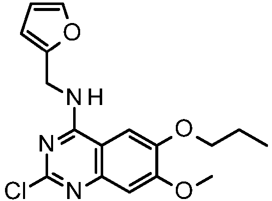 | 1H-NMR (DMSO-D6) δ: 1.01 (3H, t, J = 7.2 Hz), 1.81 (2H, m), 3.90 (3H, s), 4.01 (2H, t, J = 7.2 Hz), 1.72 (2H, d, J = 5.2 Hz), 6.37 (1H, dd, J = 3.2, 0.8 Hz), 6.43 (1H, dd, J = 3.2, 1.6 Hz), 7.09 (1H, s), 7.62 (1H, dd, J = 2.0, 0.8 Hz), 7.65 (1H, s), 8.79 (1H, t, J = 5.6 Hz). |
| | | MS (m/z): 348 (M+H)⁺. |
| 16 | 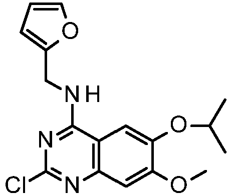 | 1H-NMR (DMSO-D6) δ: 1.32 (6H, d, J = 6.0 Hz), 3.88 (3H, s), 4.71-4.77 (3H, m), 6.37 (1H, d, J = 3.2 Hz), 6.43 (1H, dd, J = 3.2, 2.0 Hz), 7.09 (1H, s), 7.62 (1H, s), 7.68 (1H, s), 8.79 (1H, t, J = 5.2 Hz). |
| | | MS (m/z): 348 (M+H)⁺. |
| 17 | 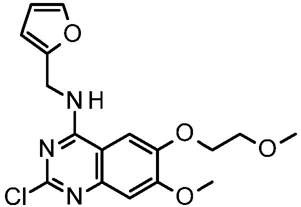 | 1H-NMR (DMSO-D6) δ: 3.33 (3H, s), 3.73 (2H, dd, J = 6.0, 4.4 Hz), 3.90 (3H, s), 4.16-4.19 (2H, m), 4.72 (2H, d, J = 5.6 Hz), 6.38 (1H, d, J = 0.4 Hz), 6.43 (1H, dd, J = 3.2, 2.0 Hz), 7.10 (1H, s), 7.62 (1H, dd, 2.0, 0.8 Hz), 7.68 (1H, s), 8.77 (1H, t, J = 5.6 Hz). |
| | | MS (m/z): 364 (M+H)⁺. |
| 18 | 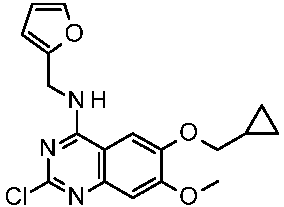 | 1H-NMR (DMSO-D6) δ: 0.33-0.37 (2H, m), 0.60-0.64 (2H, m), 1.23-1.35 (1H, m), 3.88-3.90 (5H, m), 4.71 (2H, d, J = 5.6 Hz), 6.37 (1H, d, J = 0.8 Hz), 6.42 (1H, dd, J = 3.2, 2.0 Hz), 7.09 (1H, s), 7.61-7.62 (2H, m), 8.76 (1H, t, J = 5.6 Hz). |
| | | MS (m/z): 360 (M+H)⁺. |
| 19 | 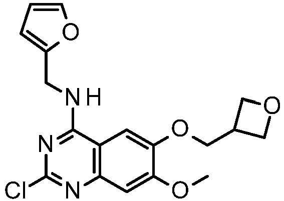 | 1H-NMR (DMSO-D6) δ: 3.42-3.50 (1H, m), 3.89 (3H, s), 4.31 (2H, d, J = 6.8 Hz), 4.42 (2H, t, J = 12 Hz), 4.73-4.78 (4H, m), 6.38 (1H, d, J = 0.8 Hz), 6.43 (1H, dd, J = 3.2, 2.0 Hz), 7.11 (1H, s), 7.62-7.63 (1H, m), 7.74 (1H, s), 8.80 (1H, t, J = 5.6 Hz). |
| | | MS (m/z): 376 (M+H)⁺. |
| 20 | 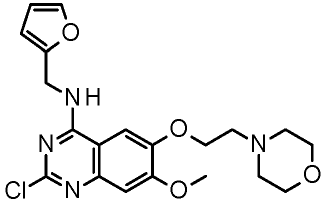 | 1H-NMR (CDCl3) δ: 2.59-2.61 (4H, m), 2.89 (2H, t, J = 6.0 Hz), 3.71-3.74 (4H, m), 3.95 (3H, s), 4.22 (2H, t, J = 6.0 Hz), 4.84 (2H, d, J = 4.8 Hz), 5.84 (1H, t, J = 4.8 Hz), 6.39-6.41 (2H, m), 6.94 (1H, s), 7.14 (1H, s), 7.43-7.44 (1H, m). |
| | | MS (m/z): 419 (M+H)⁺. |
| 21 | 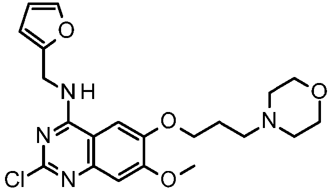 | 1H-NMR (DMSO-D6) δ: 1.90-1.99 (2H, m), 2.37 (4H, br s), 4.40 (2H, t, J = 6.4 Hz), 3.56-3.58 (4H, m), 3.89 (3H, s), 4.08 (2H, t, J = 6.4 Hz), 4.71 (2H, d, J = 5.2 Hz), 6.37 (1H, dd, J = 3.2, 0.8 Hz), 6.43 (1H, dd, J = 3.2, 1.6 Hz), 7.09 (1H, s), |
| 22 | 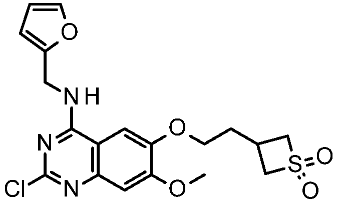 | 1H-NMR (DMSO-D6) δ: 2.14-2.19 (2H, m), 2.64-2.75 (1H, m), 3.90 (3H, s), 3.97-4.02 (2H, m), 4.08 (2H, t, J = 6.0 Hz), 4.26-4.32 (2H, m), 4.72 (2H, d, J = 5.6 Hz), 6.37 (1H, dd, J = 3.2, 0.8 Hz), 6.43 (1H, dd, J = 3.2, 2.0 Hz), 7.10 (1H, s), 7.62 (1H, dd, J = 2.0, 0.8 Hz), 7.67 (1H, s), 8.79 (1H, t, J = 5.6 Hz). |
| | | MS (m/z): 438 (M+H)⁺. |
| 23 | 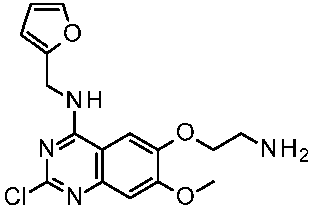 | 1H-NMR (DMSO-D6) δ: 2.95 (2H, t, J = 6.0 Hz), 3.90 (3H, s), 4.00 (2H, t, J = 6.0 Hz), 4.71 (2H, d, J = 5.2 Hz), 6.37 (1H, dd, J = 3.2, 0.4 Hz), 6.43 (1H, dd, J = 3.2, 2.0 Hz), 7.10 (1H, s), 7.62 (1H, dd, 2.0, 0.8 Hz), 7.68 (1H, s), 8.82 (1H, t, J = 5.6 Hz). |
| | | MS (m/z): 349 (M+H)⁺. |
| 24 | 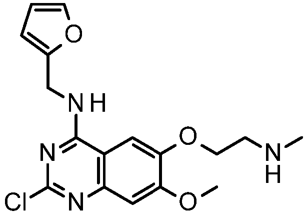 | 1H-NMR (DMSO-D6) δ: 2.36 (3H, s), 2.90 (2H, t, J = 2.0 Hz), 3.90 (3H, s), 4.09 (2H, t, J = 2.0 Hz), 4.72 (2H, d, J = 5.2 Hz), 6.37-6.38 (1H, m), 6.43 (1H, dd, J = 3.2, 2.0 Hz), 7.10 (1H, s), 7.62 (1H, s), 7.69 (1H, s), 8.80 (1H, t, J = 5.6 Hz). |
| | | MS (m/z): 363 (M+H)⁺. |
| 25 | 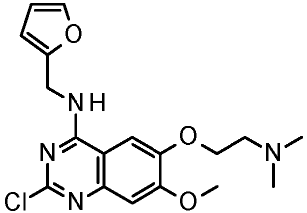 | 1H-NMR (DMSO-D6) δ: 2.24 (6H, s), 2.69 (2H, t, J = 6.0 Hz), 3.89 (3H, s), 4.12 (2H, t, J = 6.0 Hz), 4.72 (2H, d, J = 5.6 Hz), 6.37 (1H, dd, J = 3.6, 0.8 Hz), 6.43 (1H, dd, J = 3.6, 2.0 Hz), 7.09 (1H, s), 7.62 (1H, dd, 2.0, 0.8 Hz), 7.68 (1H, s), 8.78 (1H, t, J = 5.6 Hz). |
| | | MS (m/z): 377 (M+H)⁺. |
| 26 | 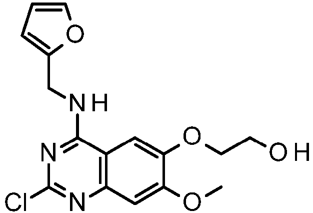 | 1H-NMR (DMSO-D6) δ: 3.79 (2H, q, J = 5.2 Hz), 3.90 (3H, s), 4.07 (2H, t, J = 5.2 Hz), 4.72 (2H, d, J = 5.6 Hz), 4.96 (1H, t, J = 5.2 Hz), 6.37-6.38 (1H, m), 6.43 (1H, dd, J = 3.2, 2.0 Hz), 7.10 (1H, s), 7.62 (1H, dd, J = 1.6, 0.8 Hz), 7.67 (1H, s), 8.80 (1H, t, J = 5.2 Hz). |
| | | MS (m/z): 350 (M+H)⁺. |
| 27 | 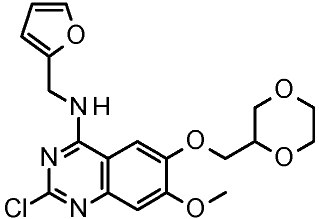 | 1H-NMR (DMSO-D6) δ: 3.42 (1H, dd, J = 11.2, 9.6 Hz), 3.48-3.55 (1H, m), 3.63-3.70 (2H, m), 3.77-3.80 (1H, m), 3.86-4.06 (7H, m), 4.72 (2H, d, J = 5.6 Hz), 6.37 (1H, d, J = 0.8 Hz), 6.43 (1H, dd, J = 3.2, 2.0 Hz), 7.11 (1H, s), 7.62 (1H, dd, J = 2.0, 0.8 Hz), 7.67 (1H, s), 8.78 (1H, t, J = 5.6 Hz). |
| | | MS (m/z): 406 (M+H)⁺. |
| 28 | 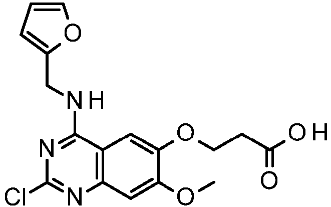 | 1H-NMR (DMSO-D6) δ: 2.79 (2H, t, J = 6.4 Hz), 3.88 (3H, s), 4.25 (2H, t, J = 6.4 Hz), 4.72 (2H, d, J = 5.6 Hz), 6.37 (1H, d, J = 0.8 Hz), 6.43 (1H, dd, J = 3.2, 2.0 Hz), 7.09 (1H, s), 7.62 (1H, dd, J = 2.0, 0.8 Hz), 7.70 (1H, s), 8.84 (1H, t, J = 5.6 Hz), 12.45 (1H, br s). |
| | | MS (m/z): 378 (M+H)⁺. |
| 29 | 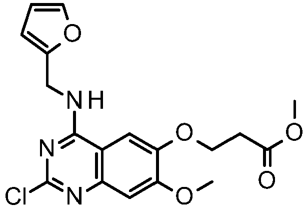 | 1H-NMR (DMSO-D6) δ: 2.90 (2H, t, J = 6.0 Hz), 3.65 (3H, s), 3.88 (3H, s), 4.29 (2H, t, J = 6.0 Hz), 4.72 (2H, d, J = 5.2 Hz), 6.37 (1H, dd, J = 3.2, 0.8 Hz), 6.43 (1H, dd, J = 3.2, 1.6 Hz), 7.10 (1H, s), 7.62 (1H, dd, J = 1.6, 0.8 Hz), 7.71 (1H, s), 8.83 (1H, t, J = 5.2 Hz). |
| | | MS (m/z): 392 (M+H)⁺. |
| 30 | 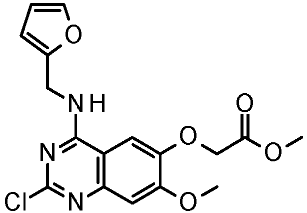 | 1H-NMR (DMSO-D6) δ: 3.72 (3H, s), 3.92 (3H, s), 4.72 (2H, d, J = 5.6 Hz), 4.89 (2H, s), 6.37 (1H, dd, J = 3.2, 0.8 Hz), 6.43 (1H, dd, J = 3.2, 2.0 Hz), 7.13 (1H, s), 7.62 (1H, dd, J = 2.0, 0.8 Hz), 7.66 (1H, s), 8.76 (1H, t, J = 5.6 Hz). |
| | | MS (m/z): 378 (M+H)⁺. |
| 31-1 | 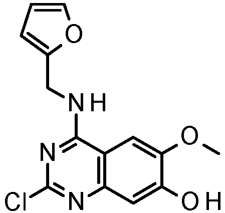 | 1H-NMR (DMSO-D6) δ: 3.88 (3H, s), 4.71 (2H, d, J = 5.6 Hz), 6.36 (1H, dd, J = 3.2, 0.8 Hz), 6.42 (1H, dd, J = 3.2, 1.6 Hz), 6.91 (1H, s), 7.61 (1H, dd, J = 1.6, 0.8 Hz), 7.65 (1H, s), 8.72 (1H, t, J = 5.6 Hz), 10.43 (1H, br s). |
| 31-2 | 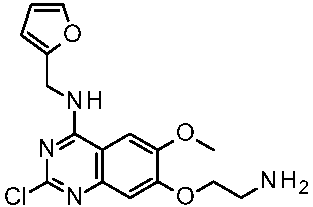 | 1H-NMR (DMSO-D6) δ: 2.93 (2H, t, J = 5.6 Hz), 3.88 (3H, s), 4.05 (2H, t, J = 5.6 Hz), 4.72 (2H, d, J = 5.2 Hz), 6.37 (1H, dd, J = 3.2, 0.8 Hz), 6.43 (1H, dd, J = 3.2, 2.0 Hz), 7.09 (1H, s), 7.62 (1H, dd, J = 1.6, 0.8 Hz), 7.67 (1H, s), 8.80 (1H, t, J = 5.2 Hz). |
| | | MS (m/z): 349 (M+H)⁺. |
| 32 | 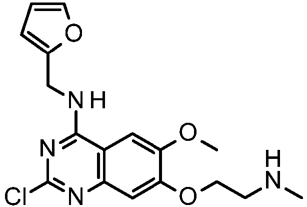 | 1H-NMR (DMSO-D6) δ: 2.34 (3H, s), 2.88 (2H, t, J = 5.6 Hz), 3.87 (3H, s), 4.14 (2H, t, J = 5.6 Hz), 4.72 (2H, d, J = 5.2 Hz), 6.37 (1H, dd, J = 3.2, 0.8 Hz), 6.43 (1H, dd, J = 3.2, 2.0 Hz), 7.10 (1H, s), 7.61 (1H, dd, J = 2.0, 0.8 Hz), 7.67 (1H, s), 8.80 (1H, t, J = 5.2 Hz). |
| | | MS (m/z): 363 (M+H)⁺. |
| 33 | 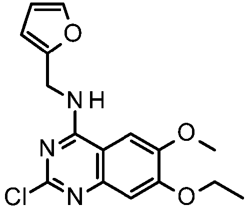 | 1H-NMR (DMSO-D6) δ: 1.38 (3H, t, J = 6.8 Hz), 3.87 (3H, s), 4.15 (2H, q, J = 6.8 Hz), 4.72 (2H, d, J = 5.2 Hz), 6.37 (1H, dd, J = 3.2, 0.8 Hz), 6.43 (1H, dd, J = 3.2, 1.6 Hz), 7.07 (1H, s), 7.62 (1H, dd, J = 1.6, 0.8 Hz), 7.66 (1H, s), 7.80 (1H, t, J = 5.2 Hz). |
| | | MS (m/z): 334 (M+H)⁺. |

### Confirmation of Effect of Suppressing Aberrant Splicing (Exon Skipping) Caused by IVS20+6T>C Mutation

A SPREADD reporter system for a splicing mutation of the IKBKAP gene in familial dysautonomia was prepared, as illustrated in FIG. 1. In this reporter system, GFP is expressed when the gene undergoes normal splicing, while RFP is expressed when the gene undergoes abnormal splicing (exon skipping).

HeLa cells transfected with the prepared SPREADD reporter construct were brought into contact with the compounds listed in Table 2 below and cultured (concentrations: 22.86237 nM, 68.58711 nM, 205.7613 nM, 617.284 nM, 1851.852 nM, 5555.556 nM, 16666.67 nM, or 50000 nM). One day later, fluorescence was measured using All-in-One Fluorescence Microscope BZ-X700 (Keyence Corp.), and the IKBKAP-FD exon 20 inclusion rate was quantified by the GFP/RFP ratio of the IKBKAP-FD reporter. Table 2 below shows the 50% effective concentration (EC₅₀). The EC₅₀ was determined with %GFP of 20 pM 2-chloro-6-(2-furylmethyl)purine set to 100%. Regression analysis for determining EC₅₀ was performed using GraphPad Prism 7.05.

**[Table 2]**

| Example | EC₅₀(µM) |
|---|---|
| 1 | 2.10 |
| 2 | 2.12 |
| 3 | 2.82 |
| 4 | 35.9 |
| 5 | 4.2 |
| 6 | 5.70 |
| 7 | 21.89 |
| 8 | 20.04 |
| 9 | 3.34 |
| 10 | 2.90 |
| 11 | 8 |
| 12 | 1.08 |
| 13 | 4.39 |
| 14 | 1.37 |
| 15 | 0.60 |
| 16 | 2.49 |
| 17 | 3.06 |
| 18 | 1.60 |
| 19 | 0.17 |
| 20 | 8.34 |
| 21 | 8.5 |
| 22 | 14.5 |
| 23 | 0.53 |
| 24 | 0.48 |
| 25 | 0.79 |
| 26 | 0.82 |
| 27 | 2.02 |
| 28 | >50 |
| 29 | 6.41 |
| 30 | 26.41 |
| 31 | 3.15 |
| 32 | 4.29 |
| 33 | 3.65 |

The above-listed compounds demonstrated higher expression levels of GFP than RFP, confirming their effect of suppressing abnormal splicing (exon skipping) caused by the IVS20^{+6T>C} mutation.

As shown in Table 2, compounds were obtained that had a greater effect of suppressing abnormal splicing than the known compound (2-chloro-6-(2-furylmethyl)purine), which has been found to be able to correct abnormal splicing and treat FD.

## Claims

1. A compound represented by Formula (I) below or a pharmaceutically acceptable salt thereof: 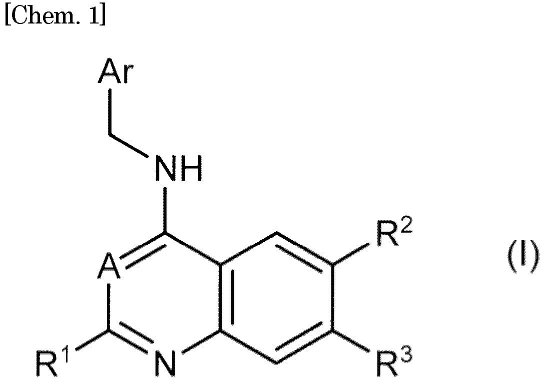 wherein, in Formula (I),
A represents CH or N;
R¹ represents a halogen atom;
R² and R³ are each independently selected from the group consisting of a hydrogen atom, -OR⁴, -O(CH₂)ₜR⁵, and -O(CH₂)ₜC(O)R⁵, where at least one of R² and R³ represents -OR⁴, -O(CH₂)ₜR⁵, or -O(CH₂)ₜC(O)R⁵, and t is 1, 2, 3, or 4,
R⁴ representing a hydrogen atom or a C₁-C₆ alkyl group, and
R⁵ being selected from the group consisting of a C₃-C₆ cycloalkyl group, a 4- to 10-membered heterocyclyl group, -NR⁶R⁷, and -OR⁶,
R⁶ and R⁷ each independently representing a hydrogen atom or a C₁-Cs alkyl group; and
Ar is selected from the group consisting of a 5- to 10-membered heteroaryl group and a 6- to 12-membered aryl group, where the 5- to 10-membered heteroaryl group and the 6- to 12-membered aryl group may have one or more substituents.

2. The compound according to claim 1 or pharmaceutically acceptable salt thereof,
wherein R³ represents a methoxy group (-OCH₃).

3. The compound according to claim 1 or pharmaceutically acceptable salt thereof,
wherein Ar represents a furyl group, a thiophenyl group, a pyrrolyl group, a thiazolyl group, an oxazolyl group, or a pyridyl group.

4. The compound according to claim 2 or pharmaceutically acceptable salt thereof,
wherein R² is selected from the group consisting of a hydrogen atom, -OR⁴, - O(CH₂)ₜR⁵, and -O(CH₂)ₜC(O)R⁵,
R⁴ representing a C₁-C₃ alkyl group,
R⁵ representing -NR⁶R⁷, -OH, a methyl group, a cyclopropyl group, an oxetanyl group, a morpholinyl group, a 1, 1-dioxo-thietanyl group, or a dioxanyl group,
R⁶ and R⁷ each independently representing a hydrogen atom or a methyl group, and
t being 1 or 2.

5. The compound according to claim 1 or pharmaceutically acceptable salt thereof,
wherein Ar represents a 2-furyl group,
R² represents a hydrogen atom, -O(CH₂)₂CH₃, -OCH₂R⁵, or -O(CH₂)₂R⁵, where R⁵ represents -NR⁶R⁷, -OH, or an oxetanyl group, where R⁶ and R⁷ each independently represent a hydrogen atom or a methyl group, and
R³ represents a methoxy group (-OCH₃).

6. A compound or a pharmaceutically acceptable salt thereof, the compound being any one compound selected from the group consisting of:
2-chloro-N-(furan-2-ylmethyl)-6,7-dimethoxyquinazolin-4-amine,
2-chloro-6,7-dimethoxy-N-(thiophen-2-ylmethyl)quinazolin-4-amine,
2-chloro-6,7-dimethoxy-N-(thiophen-3-ylmethyl)quinazolin-4-amine,
2-chloro-6,7-dimethoxy-N-(1,3-thiazol-2-ylmethyl)quinazolin-4-amine,
2-chloro-6,7-dimethoxy-N-(pyridin-4-ylmethyl)quinazolin-4-amine,
2-chloro-6,7-dimethoxy-N-(1,3-oxazol-2-ylmethyl)quinazolin-4-amine,
2-chloro-6,7-dimethoxy-N-(1H-pyrrol-3-ylmethyl)quinazolin-4-amine,
2-chloro-N-(furan-3-ylmethyl)-6,7-dimethoxyquinazolin-4-amine,
2-chloro-6,7-dimethoxy-N-(pyridin-2-ylmethyl)quinazolin-4-amine,
2-chloro-N-(furan-2-ylmethyl)-7-methoxyquinazolin-4-amine,
2-chloro-N-(furan-2-ylmethyl)-6-methoxyquinazolin-4-amine,
2-chloro-N-(furan-2-ylmethyl)-7-methoxyquinolin-4-amine,
2-chloro-N-(furan-2-ylmethyl)-6-methoxyquinolin-4-amine,
2-chloro-6-ethoxy-N-(furan-2-ylmethyl)-7-methoxyquinazolin-4-amine,
2-chloro-6-ethoxy-N-(furan-2-ylmethyl)-7-methoxyquinazolin-4-amine,
2-chloro-N-(furan-2-ylmethyl)-7-methoxy-6-propoxyquinazolin-4-amine,
2-chloro-N-(furan-2-ylmethyl)-7-methoxy-6-(propan-2-yloxy)quinazolin-4-amine,
2-chloro-N-(furan-2-ylmethyl)-7-methoxy-6-(2-methoxyethoxy)quinazolin-4-amine,
2-chloro-6-(cyclopropylmethoxy)-N-(furan-2-ylmethyl)-7-methoxyquinazolin-4-amine,
2-chloro-N-(furan-2-ylmethyl)-7-methoxy-6-(oxetan-3-ylmethoxy)quinazolin-4-amine,
2-chloro-N-(furan-2-ylmethyl)-7-methoxy-6-[2-(morpholin-4-yl)ethoxy] quinazolin-4-amine,
2-chloro-N-(furan-2-ylmethyl)-7-methoxy-6-[3-(morpholin-4-yl)propoxy]quinazolin-4-amine,
2-chloro-6-[2-(1,1-dioxidothiethan-3-yl)ethoxy]-N-(furan-2-ylmethyl)-7-methoxyquinazolin-4-amine,
6-(2-aminoethoxy)-2-chloro-N-(furan-2-ylmethyl)-7-methoxyquinazolin-4-amine,
2-chloro-N-(furan-2-ylmethyl)-7-methoxy-6-[2-(methylamino)ethoxy]quinazolin-4-amine,
2-chloro-6-[2-(dimethylamino)ethoxy]-N-(furan-2-ylmethyl)-7-methoxyquinazolin-4-amine,
2-({2-chloro-4-[(furan-2-ylmethyl)amino]-7-methoxyquinazolin-6-yl}oxy)ethanol,
2-chloro-6-(1,4-dioxan-2-ylmethoxy)-N-(furan-2-ylmethyl)-7-methoxyquinazolin-4-amine,
3-({2-chloro-4-[(furan-2-ylmethyl)amino]-7-methoxyquinazolin-6-yl}oxy)propanoic acid,
methyl 3-({2-chloro-4-[(furan-2-ylmethyl)amino]-7-methoxyquinazolin-6-yl}oxy)propanoate,
methyl ({2-chloro-4-[(furan-2-ylmethyl)amino]-7-methoxyquinazolin-6-yl}oxy)acetate,
7-(2-aminoethoxy)-2-chloro-N-(furan-2-ylmethyl)-6-methoxyquinazolin-4-amine,
2-chloro-4-[(furan-2-ylmethyl)amino]-6-methoxyquinazolin-7-ol,
2-chloro-N-(furan-2-ylmethyl)-6-methoxy-7-[2-(methylamino)ethoxy]quinazolin-4-amine, and
2-chloro-7-ethoxy-N-(furan-2-ylmethyl)-6-methoxyquinazolin-4-amine.

7. A pharmaceutical composition containing, as an active ingredient, the compound according to any one of claims 1 to 6 or pharmaceutically acceptable salt thereof.

8. A pharmaceutical composition for treating a genetic disease caused by aberrant splicing, the pharmaceutical composition containing, as an active ingredient, the compound according to any one of claims 1 to 6 or pharmaceutically acceptable salt thereof.

9. A pharmaceutical composition for treating a genetic disease caused by aberrant splicing,
the pharmaceutical composition containing, as an active ingredient, a compound represented by Formula (I) below or a pharmaceutically acceptable salt thereof: 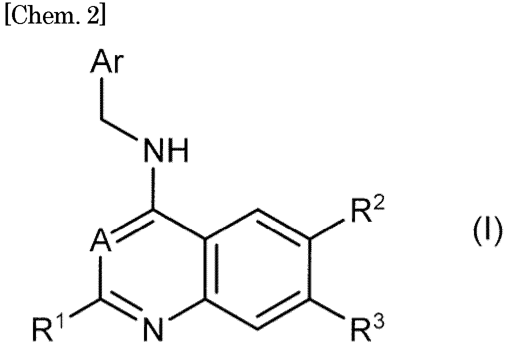 wherein, in Formula (I),
A represents CH or N;
R¹ represents a halogen atom;
R² and R³ are each independently selected from the group consisting of a hydrogen atom, -OR⁴, -O(CH₂)ₜR⁵, and -O(CH₂)ₜC(O)R⁵, where at least one of R² and R³ represents -OR⁴, -O(CH₂)ₜR⁵, or -O(CH₂)ₜC(O)R⁵, and t is 1, 2, 3, or 4,
R⁴ representing a hydrogen atom or a C₁-C₆ alkyl group, and
R⁵ being selected from the group consisting of a C₃-C₆ cycloalkyl group, a 4- to 10-membered heterocyclyl group, -NR⁶R⁷, and -OR⁶,
R⁶ and R⁷ each independently representing a hydrogen atom or a C₁-Cs alkyl group; and
Ar is selected from the group consisting of a 5- to 10-membered heteroaryl group and a 6- to 12-membered aryl group, where the 5- to 10-membered heteroaryl group and the 6- to 12-membered aryl group may have one or more substituents.

10. The pharmaceutical composition according to claim 9,
wherein Ar represents a 2-furyl group,
R² represents a hydrogen atom, -O(CH₂)₂CH₃, -OCH₂R⁵, or -O(CH₂)₂R⁵, where R⁵ represents -NR⁶R⁷, -OH, or an oxetanyl group, where R⁶ and R⁷ each independently represent a hydrogen atom or a methyl group, and
R³ represents a methoxy group (-OCH₃).

11. The pharmaceutical composition according to claim 9 or 10,
wherein the genetic disease caused by aberrant splicing is familial dysautonomia or congenital long QT syndrome.

12. A method for treating a genetic disease caused by aberrant splicing, the method comprising:
administering the compound according to any one of claims 1 to 6 or pharmaceutically acceptable salt thereof to a subject.

13. The method according to claim 12,
wherein the genetic disease caused by aberrant splicing is familial dysautonomia or congenital long QT syndrome.

14. Use of the compound according to any one of claims 1 to 6 or pharmaceutically acceptable salt thereof in production of a pharmaceutical composition for use in treatment of a genetic disease caused by aberrant splicing.

15. The use according to claim 14,
wherein the genetic disease caused by aberrant splicing is familial dysautonomia or congenital long QT syndrome.
